# EUROPEAN PATENT SPECIFICATION

(11) **EP 2 049 667 B1**
(45) Date of publication and mention of the grant of the patent: **16.03.2011**
(21) Application number: 07825556.9
(22) Date of filing: 25.07.2007
(51) Int. Cl.: C12N 15/63

(54) **RECOMBINANT MYCOBACTERIUM STRAIN EXPRESSING A MYCOBACTERIAL FAP PROTEIN UNDER THE CONTROL OF A PROMOTER ACTIVE UNDER HYPOXIA AND ITS APPLICATION FOR CANCER THERAPY**
REKOMBINANTER MYCOBAKTERIENSTAMM ZUR DURCH EINEN BEI HYPOXIE AKTIVEN PROMOTOR GESTEUERTEN EXPRESSION EINES MYKOBAKTERIELLEN FAP-PROTEINS UND SEINE ANWENDUNG ZUR KREBSTHERAPIE
SOUCHE DE MYCOBACTERIUM EXPRIMANT UNE PROTEINE FAP SOUS LE CONTRÔLE D'UN PROMOTEUR QUI EST ACTIF EN CONDITIONS D' HYPOXIE ET SES APPLICATIONS POUR LA THÉRAPIE DU CANCER

(30) Priority: 25.07.2006 EP 06291205
(43) Date of publication of application: 22.04.2009
(73) Proprietor: INSTITUT PASTEUR, 75015 Paris (FR)
(72) Inventor: MARCHAL, Gilles, 94200 Ivry-sur-Seine (FR); ABOLHASSANI, Mohammad, 75015 Paris (FR)
(74) Representative: Leblois-Préhaud, Hélène Marthe Georgette
(86) International application number: PCT/IB2007/003300
(87) International publication number: WO 2008/012693

(56) References cited:
- WO-A-96/20276
- LAQUEYRERIE A ET AL: "CLONING, SEQUENCING AND EXPRESSION OF THE APA GENE CODING FOR THE MYCOBACTERIUM TUBERCULOSIS 45/47-KILODALTON SECRETED ANTIGEN COMPLEX" INFECTION AND IMMUNITY, AMERICAN SOCIETY FOR MICROBIOLOGY. WASHINGTON, US, vol. 63, no. 10, 1 October 1995 (1995-10-01), pages 4003-4010, XP002001489 ISSN: 0019-9567
- YUAN ET AL: "Stationary phase-associated protein expression in Mycobacterium tuberculosis: function of the mycobacterial alpha-crystallin homolog" JOURNAL OF BACTERIOLOGY, WASHINGTON, DC, US, vol. 178, no. 15, August 1996 (1996-08), pages 4484-4492, XP002086106 ISSN: 0021-9193 cited in the application
- FLORCZYK MATTHEW A ET AL: "A family of acr-coregulated Mycobacterium tuberculosis genes shares a common DNA motif and requires Rv3133c (dosR or devR) for expression." INFECTION AND IMMUNITY, vol. 71, no. 9, September 2003 (2003-09), pages 5332-5343, XP002401486 ISSN: 0019-9567 cited in the application
- ZHAO WEICHENG ET AL: "Role of a bacillus Calmette-Guerin fibronectin attachment protein in BCG-induced antitumor activity" INTERNATIONAL JOURNAL OF CANCER, vol. 86, no. 1, 1 April 2000 (2000-04-01), pages 83-88, XP002401487 ISSN: 0020-7136
- LEE CHI-FENG ET AL: "Treatment of bladder carcinomas using recombinant BCG DNA vaccines and electroporative gene immunotherapy." CANCER GENE THERAPY, vol. 11, no. 3, March 2004 (2004-03), pages 194-207, XP002401488 ISSN: 0929-1903
- LOSA ET AL: "LOW DOSE BACILLUS CALMETTE-GUERIN FOR CARCINOMA IN SITU OF THE BLADDER: LONG-TERM RESULTS" JOURNAL OF UROLOGY, BALTIMORE, MD, US, vol. 163, no. 1, January 2000 (2000-01), pages 68-72, XP005556180 ISSN: 0022-5347
- FLORCZYK M A ET AL: "IDENTIFICATION AND CHARACTERIZATION OF MYCOBACTERIAL PROTEINS DIFFERENTIALLY EXPRESSED UNDER STANDING AND SHAKING CULTURE CONDITIONS, INCLUDING RV2623 FROM A NOVEL CLASS OF PUTATIVE ATP-BINDING PROTEINS" INFECTION AND IMMUNITY, AMERICAN SOCIETY FOR MICROBIOLOGY. WASHINGTON, US, vol. 69, no. 9, September 2001 (2001-09), pages 5777-5785, XP001086502 ISSN: 0019-9567

### SEQUENCE LISTING

<110> INSTITUT PASTEUR
   MARCHAL, Gilles
   ABOLHASSANI, Mohammad
<120> RECOMBINANT MYCOBACTERIUM STRAIN EXPRESSING A MYCOBACTERIAL FAP PROTEIN UNDER THE CONTROL OF A PROMOTER ACTIVE UNDER HYPOXIA AND ITS APPLICATION FOR CANCER THERAPY
<130> BLO/HLP/cp226-131
<160> 19
<170> PatentIn version 3.3
<210> 1
   <211> 33
   <212> DNA
   <213> Artificial
<220>
   <223> Forward primer
<400> 1
   catgccatgg tacaggtgga ccccaacttg aca 33
<210> 2
   <211> 33
   <212> DNA
   <213> Artificial
<220>
   <223> Reverse primer
<400> 2
   ttaggatccg gccggtaagg tccgctgcgg tgt 33
<210> 3
   <211> 29
   <212> DNA
   <213> Artificial
<220>
   <223> Bamhspx R primer
<400> 3
   ttaggatccg tccggcatga tcaacctcc 29
<210> 4
   <211> 30
   <212> DNA
   <213> Artificial
<220>
   <223> NcohspX F primer
<400> 4
   catgccatgg ggtggccatt tgatgcctcc 30
<210> 5
   <211> 29
   <212> DNA
   <213> Artificial
<220>
   <223> BamRv2623 F primer
<400> 5
   ttaggatccg ggccatggac tggtcgtcg 29
<210> 6
   <211> 30
   <212> DNA
   <213> Artificial
<220>
   <223> NcoRV2623 R primer
<400> 6
   catgccatgg acatcgcggt cctcctgtcg 30
<210> 7
   <211> 25
   <212> DNA
   <213> artificial sequence
<220>
   <223> sma15up primer
<400> 7
   tcccccgggg gtgttgaccc gacac 25
<210> 8
   <211> 23
   <212> DNA
   <213> artificial sequence
<220>
   <223> Pst23up primer
<400> 8
   aactgcaggg cgaagaacct acc 23
<210> 9
   <211> 23
   <212> DNA
   <213> artificial sequence
<220>
   <223> Pst35down primer
<400> 9
   aactgcagcc aagtgatacc cct 23
<210> 10
   <211> 24
   <212> DNA
   <213> artificial sequence
<220>
   <223> Hin43down primer
<400> 10
   cccaagcttg gagatcggtg cggc 24
<210> 11
   <211> 2061
   <212> DNA
   <213> Mycobacterium tuberculosis
<220>
   <221> CDS
   <222> (1082)..(2059)
<400> 11
   gtgctcgggc ccaacggtgc gggcaagtcc accgccctgc atgttatcgc ggggctgctt     60
   cgcccccgac gcgggcttgg tacgtttggg ggaccgggtg ttgaccgaca ccgaggccgg    120
   ggtgaatgtg gcgacccacg accgtcgagt cgggctgctg ttgcaagacc cgttgttgtt    180
   tccacacctg agcgtggcca aaaacgtggc cttcggacca caatgccgtc gcgggatgtt    240
   tgggtccggg cgcgcgctag gacaagggcg tcggcactgc gatggctgcg cgaggtgaac    300
   gccgagcagt tcgccgaccg taagcctcgt cagctatccg ggggccaagc ccagcgcgtc    360
   gccatcgcgc gagcgttggc ggccgaaccg gatgtgttgc tgctcgacga gccgctgacc    420
   ggactcgatg tggccgcggc cgcgggtatc cgttcggtgt tgcgtagtgt cgtcgcgagg    480
   agcggttgcg cggtagtcct gacgacccat gacctgctgg acgtgttcac gctggccgac    540
   cgggtattgg tgctcgagtc cggcacgatc gccgagatcg gcccggttgc cgatgtgctt    600
   accgcacctc gcagtcgttt cggagcccgt atcgccggag tcaacctggt caatgggacc    660
   attggtccgg acggctcgct gcgcacccag tccggcgccc actggtacgg caccccggtc    720
   caggatttgc ctactgggca tgaggcaatc gcggtgttcc cgccgacggc ggtggcggtg    780
   tatccggaac cgccgcacgg aagcccgcgc aatatcgtcg ggctgacggt ggcggaggtg    840
   gatacccgcg gacccacggt cctggtgcgc gggcatgatc agcctggtgg cgcgcctggc    900
   cttgccgcat gcatcaccgt cgatgccgcc accgaactgc gtgtggcgcc cggatcgcgc    960
   gtgtggttca gcgtcaaggc gcaggaagtg gccctgcacc cggcacccca ccaacacgcc   1020
   agttcatgag ccgacccgcg ccgtccttgc gtcgcgccgt taacacggta ggttcttcgc   1080
   c atg cat cag gtg gac ccc aac ttg aca cgt cgc aag gga cga ttg gcg   1129
     Met His Gln Val Asp Pro Asn Leu Thr Arg Arg Lys Gly Arg Leu Ala
     1               5                   10                  15
   gca ctg gct atc gcg gcg atg gcc agc gcc agc ctg gtg acc gtt gcg     1177
   Ala Leu Ala Ile Ala Ala Met Ala Ser Ala Ser Leu Val Thr Val Ala
               20                  25                  30
   gtg ccc gcg acc gcc aac gcc gat ccg gag cca gcg ccc ccg gta ccc     1225
   Val Pro Ala Thr Ala Asn Ala Asp Pro Glu Pro Ala Pro Pro Val Pro
           35                  40                  45
   aca acg gcc gcc tcg ccg ccg tcg acc gct gca gcg cca ccc gca ccg     1273
   Thr Thr Ala Ala Ser Pro Pro Ser Thr Ala Ala Ala Pro Pro Ala Pro
       50                  55                  60
   gcg aca cct gtt gcc ccc cca cca ccg gcc gcc gcc aac acg ccg aat     1321
   Ala Thr Pro Val Ala Pro Pro Pro Pro Ala Ala Ala Asn Thr Pro Asn
   65                  70                  75                  80
   gcc cag ccg ggc gat ccc aac gca gca cct ccg ccg gcc gac ccg aac     1369
   Ala Gln Pro Gly Asp Pro Asn Ala Ala Pro Pro Pro Ala Asp Pro Asn
                   85                  90                  95
   gca ccg ccg cca cct gtc att gcc cca aac gca ccc caa cct gtc cgg      1417
   Ala Pro Pro Pro Pro Val Ile Ala Pro Asn Ala Pro Gln Pro Val Arg
               100                 105                 110
   atc gac aac ccg gtt gga gga ttc agc ttc gcg ctg cct gct ggc tgg      1465
   Ile Asp Asn Pro Val Gly Gly Phe Ser Phe Ala Leu Pro Ala Gly Trp
           115                 120                 125
   gtg gag tct gac gcc gcc cac ttc gac tac ggt tca gca ctc ctc agc      1513
   Val Glu Ser Asp Ala Ala His Phe Asp Tyr Gly Ser Ala Leu Leu Ser
       130                 135                 140
   aaa acc acc ggg gac ccg cca ttt ccc gga cag ccg ccg ccg gtg gcc      1561
   Lys Thr Thr Gly Asp Pro Pro Phe Pro Gly Gln Pro Pro Pro Val Ala
   145                 150                 155                 160
   aat gac acc cgt atc gtg ctc ggc cgg cta gac caa aag ctt tac gcc      1609
   Asn Asp Thr Arg Ile Val Leu Gly Arg Leu Asp Gln Lys Leu Tyr Ala
                   165                 170                 175
   agc gcc gaa gcc acc gac tcc aag gcc gcg gcc cgg ttg ggc tcg gac      1657
   Ser Ala Glu Ala Thr Asp Ser Lys Ala Ala Ala Arg Leu Gly Ser Asp
               180                 185                 190
   atg ggt gag ttc tat atg ccc tac ccg ggc acc cgg atc aac cag gaa      1705
   Met Gly Glu Phe Tyr Met Pro Tyr Pro Gly Thr Arg Ile Asn Gln Glu
           195                 200                 205
   acc gtc tcg ctc gac gcc aac ggg gtg tct gga agc gcg tcg tat tac      1753
   Thr Val Ser Leu Asp Ala Asn Gly Val Ser Gly Ser Ala Ser Tyr Tyr
       210                 215                 220
   gaa gtc aag ttc agc gat ccg agt aag ccg aac ggc cag atc tgg acg      1801
   Glu Val Lys Phe Ser Asp Pro Ser Lys Pro Asn Gly Gln Ile Trp Thr
   225                 230                 235                 240
   ggc gta atc ggc tcg ccc gcg gcg aac gca ccg gac gcc ggg ccc cct      1849
   Gly Val Ile Gly Ser Pro Ala Ala Asn Ala Pro Asp Ala Gly Pro Pro
                   245                 250                 255
   cag cgc tgg ttt gtg gta tgg ctc ggg acc gcc aac aac ccg gtg gac      1897
   Gln Arg Trp Phe Val Val Trp Leu Gly Thr Ala Asn Asn Pro Val Asp
               260                 265                 270
   aag ggc gcg gcc aag gcg ctg gcc gaa tcg atc cgg cct ttg gtc gcc      1945
   Lys Gly Ala Ala Lys Ala Leu Ala Glu Ser Ile Arg Pro Leu Val Ala
           275                 280                 285
   ccg ccg ccg gcg ccg gca ccg gct cct gca gag ccc gct ccg gcg ccg      1993
   Pro Pro Pro Ala Pro Ala Pro Ala Pro Ala Glu Pro Ala Pro Ala Pro
       290                 295                 300
   gcg ccg gcc ggg gaa gtc gct cct acc ccg acg aca ccg aca ccg cag      2041
   Ala Pro Ala Gly Glu Val Ala Pro Thr Pro Thr Thr Pro Thr Pro Gln
   305                 310                 315                 320
   cgg acc tta ccg gcc tga cc                                           2061
   Arg Thr Leu Pro Ala
                   325
<210> 12
   <211> 325
   <212> PRT
   <213> Mycobacterium tuberculosis
<400> 12
   Met His Gln Val Asp Pro Asn Leu Thr Arg Arg Lys Gly Arg Leu Ala
   1               5                   10                  15
   Ala Leu Ala Ile Ala Ala Met Ala Ser Ala Ser Leu Val Thr Val Ala
               20                  25                  30
   Val Pro Ala Thr Ala Asn Ala Asp Pro Glu Pro Ala Pro Pro Val Pro
           35                  40                  45
   Thr Thr Ala Ala Ser Pro Pro Ser Thr Ala Ala Ala Pro Pro Ala Pro
       50                  55                  60
   Ala Thr Pro Val Ala Pro Pro Pro Pro Ala Ala Ala Asn Thr Pro Asn
   65                  70                  75                  80
   Ala Gln Pro Gly Asp Pro Asn Ala Ala Pro Pro Pro Ala Asp Pro Asn
                   85                  90                  95
   Ala Pro Pro Pro Pro Val Ile Ala Pro Asn Ala Pro Gln Pro Val Arg
               100                 105                 110
   Ile Asp Asn Pro Val Gly Gly Phe Ser Phe Ala Leu Pro Ala Gly Trp
           115                 120                 125
   Val Glu Ser Asp Ala Ala His Phe Asp Tyr Gly Ser Ala Leu Leu Ser
       130                 135                 140
   Lys Thr Thr Gly Asp Pro Pro Phe Pro Gly Gln Pro Pro Pro Val Ala
   145                 150                 155                 160
   Asn Asp Thr Arg Ile Val Leu Gly Arg Leu Asp Gln Lys Leu Tyr Ala
                   165                 170                 175
   Ser Ala Glu Ala Thr Asp Ser Lys Ala Ala Ala Arg Leu Gly Ser Asp
               180                 185                 190
   Met Gly Glu Phe Tyr Met Pro Tyr Pro Gly Thr Arg Ile Asn Gln Glu
           195                 200                 205
   Thr Val Ser Leu Asp Ala Asn Gly Val Ser Gly Ser Ala Ser Tyr Tyr
       210                 215                 220
   Glu Val Lys Phe Ser Asp Pro Ser Lys Pro Asn Gly Gln Ile Trp Thr
   225                 230                 235                 240
   Gly Val Ile Gly Ser Pro Ala Ala Asn Ala Pro Asp Ala Gly Pro Pro
                   245                 250                 255
   Gln Arg Trp Phe Val Val Trp Leu Gly Thr Ala Asn Asn Pro Val Asp
   260 265 270
   Lys Gly Ala Ala Lys Ala Leu Ala Glu Ser Ile Arg Pro Leu Val Ala
   275 280 285
   Pro Pro Pro Ala Pro Ala Pro Ala Pro Ala Glu Pro Ala Pro Ala Pro
   290 295 300
   Ala Pro Ala Gly Glu Val Ala Pro Thr Pro Thr Thr Pro Thr Pro Gln
   305 310 315 320
   Arg Thr Leu Pro Ala
   325
<210> 13
   <211> 214
   <212> DNA
   <213> Mycobacterium bovis
<400> 13
   caccatggtg tccggcatga tcaacctccg ctgttcgata tcaccccgat ctttctgaac 60
   ggcggttggc agacaacagg gtcaatggtc cccaagtgga tcaccgacgg gcgcggacaa 120
   atggcccgcg cttcggggac ttctgtccct agccctggcc acgatgggct ggtcggatca 180
   aaggcatccg tttccatcga ttaggaggca tcaa 214
<210> 14
   <211> 371
   <212> DNA
   <213> Mycobacterium tuberculosis
<400> 14
   gggccatgga ctggtcgtcg aacacgttgt caccgcttcc atggcgttgt tacaaccgcg 60
   acgggtgatc gctgacgaag gcctcctggg tgcgctgcgg ttcgccggaa acctgctcat 120
   ccatcgtgcc gcgcgtcggc gagtcctgtt gatgcgccac acattccgca ggcatcgtga 180
   acgcttgaca gccgtcgcca ttgtcgcgca caaaccgcac gtcgattcgt gatccattga 240
   ggacctaagc ccgttgggct agtgacaaac gcctcctgag caaaaccctc ctcccccgtt 300
   accgtcgtgc ggtagggaca agccacatcg gccgagcggg cgatcagcca acgacaggag 360
   gaccgcgatg t 371
<210> 15
   <211> 990
   <212> DNA
   <213> artificial sequence
<220>
   <223> apa NcoI-BamHI fragment
<400> 15
   catgccatgg tacaggtgga ccccaacttg acacgtcgca agggacgatt ggcggcactg     60
   gctatcgcgg cgatggccag cgccagcctg gtgaccgttg cggtgcccgc gaccgccaac    120
   gccgatccgg agccagcgcc cccggtaccc acaacggccg cctcgccgcc gtcgaccgct    180
   gcagcgccac ccgcaccggc gacacctgtt gcccccccac caccggccgc cgccaacacg    240
   ccgaatgccc agccgggcga tcccaacgca gcacctccgc cggccgaccc gaacgcaccg    300
   ccgccacctg tcattgcccc aaacgcaccc caacctgtcc ggatcgacaa cccggttgga    360
   ggattcagct tcgcgctgcc tgctggctgg gtggagtctg acgccgccca cttcgactac    420
   ggttcagcac tcctcagcaa aaccaccggg gacccgccat ttcccggaca gccgccgccg    480
   gtggccaatg acacccgtat cgtgctcggc cggctagacc aaaagcttta cgccagcgcc    540
   gaagccaccg actccaaggc cgcggcccgg ttgggctcgg acatgggtga gttctatatg    600
   ccctacccgg gcacccggat caaccaggaa accgtctcgc tcgacgccaa cggggtgtct    660
   ggaagcgcgt cgtattacga agtcaagttc agcgatccga gtaagccgaa cggccagatc    720
   tggacgggcg taatcggctc gcccgcggcg aacgcaccgg acgccgggcc ccctcagcgc    780
   tggtttgtgg tatggctcgg gaccgccaac aacccggtgg acaagggcgc ggccaaggcg    840
   ctggccgaat cgatccggcc tttggtcgcc ccgccgccgg cgccggcacc ggctcctgca    900
   gagcccgctc cggcgccggc gccggccggg gaagtcgctc ctaccccgac gacaccgaca    960
   ccgcagcgga ccttaccggc cggatcctaa                                     990
<210> 16
   <211> 233
   <212> DNA
   <213> artificial sequence
<220>
   <223> acr/hspX promoter BamHI-NcoI fragment
<400> 16
   ttaggatccg tccggcatga tcaacctccg ctgttcgata tcaccccgat ctttctgaac     60
   ggcggttggc agacaacagg gtcaatggtc cccaagtgga tcaccgacgg gcgcggacaa    120
   atggcccgcg cttcggggac ttctgtccct agccctggcc acgatgggct ggtcggatca    180
   aaggcatccg tttccatcga ttaggaggca tcaaatggcc accccatggc atg           233
<210> 17
   <211> 390
   <212> DNA
   <213> artificial sequence
<220>
   <223> Rv2623 promoter BamHI-NCOI fragment
<400> 17
   ttaggatccg ggccatggac tggtcgtcga acacgttgtc accgcttcca tggcgttgtt     60
   acaaccgcga cgggtgatcg ctgacgaagg cctcctgggt gcgctgcggt tcgccggaaa    120
   cctgctcatc catcgtgccg cgcgtcggcg agtcctgttg atgcgccaca cattccgcag    180
   gcatcgtgaa cgcttgacag ccgtcgccat tgtcgcgcac aaaccgcacg tcgattcgtg    240
   atccattgag gacctaagcc cgttgggcta gtgacaaacg cctcctgagc aaaaccctcc    300
   tcccccgtta ccgtcgtgcg gtagggacaa gccacatcgg ccgagcgggc gatcagccaa    360
   cgacaggagg accgcgatgt ccatggcatg                                     390
<210> 18
   <211> 1201
   <212> DNA
   <213> artificial sequence
<220>
   <223> acr-apa cassette
<400> 18
   gatccgtccg gcatgatcaa cctccgctgt tcgatatcac cccgatcttt ctgaacggcg     60
   gttggcagac aacagggtca atggtcccca agtggatcac cgacgggcgc ggacaaatgg    120
   cccgcgcttc ggggacttct gtccctagcc ctggccacga tgggctggtc ggatcaaagg    180
   catccgtttc catcgattag gaggcatcaa atggccaccc catggtacag gtggacccca    240
   acttgacacg tcgcaaggga cgattggcgg cactggctat cgcggcgatg gccagcgcca    300
   gcctggtgac cgttgcggtg cccgcgaccg ccaacgccga tccggagcca gcgcccccgg    360
   tacccacaac ggccgcctcg ccgccgtcga ccgctgcagc gccacccgca ccggcgacac    420
   ctgttgcccc cccaccaccg gccgccgcca acacgccgaa tgcccagccg ggcgatccca    480
   acgcagcacc tccgccggcc gacccgaacg caccgccgcc acctgtcatt gccccaaacg    540
   caccccaacc tgtccggatc gacaacccgg ttggaggatt cagcttcgcg ctgcctgctg    600
   gctgggtgga gtctgacgcc gcccacttcg actacggttc agcactcctc agcaaaacca    660
   ccggggaccc gccatttccc ggacagccgc cgccggtggc caatgacacc cgtatcgtgc    720
   tcggccggct agaccaaaag ctttacgcca gcgccgaagc caccgactcc aaggccgcgg    780
   cccggttggg ctcggacatg ggtgagttct atatgcccta cccgggcacc cggatcaacc    840
   aggaaaccgt ctcgctcgac gccaacgggg tgtctggaag cgcgtcgtat tacgaagtca    900
   agttcagcga tccgagtaag ccgaacggcc agatctggac gggcgtaatc ggctcgcccg    960
   cggcgaacgc accggacgcc gggccccctc agcgctggtt tgtggtatgg ctcgggaccg    1020
   ccaacaaccc ggtggacaag ggcgcggcca aggcgctggc cgaatcgatc cggcctttgg   1080
   tcgccccgcc gccggcgccg gcaccggctc ctgcagagcc cgctccggcg ccggcgccgg   1140
   ccggggaagt cgctcctacc ccgacgacac cgacaccgca gcggacctta ccggccggat   1200
   c                                                                   1201
<210> 19
   <211> 1358
   <212> DNA
   <213> artificial sequence
<220>
   <223> Rv2623-apa cassette
<400> 19
   gatccgggcc atggactggt cgtcgaacac gttgtcaccg cttccatggc gttgttacaa     60
   ccgcgacggg tgatcgctga cgaaggcctc ctgggtgcgc tgcggttcgc cggaaacctg    120
   ctcatccatc gtgccgcgcg tcggcgagtc ctgttgatgc gccacacatt ccgcaggcat    180
   cgtgaacgct tgacagccgt cgccattgtc gcgcacaaac cgcacgtcga ttcgtgatcc    240
   attgaggacc taagcccgtt gggctagtga caaacgcctc ctgagcaaaa ccctcctccc    300
   ccgttaccgt cgtgcggtag ggacaagcca catcggccga gcgggcgatc agccaacgac    360
   aggaggaccg cgatgtccat ggtacaggtg gaccccaact tgacacgtcg caagggacga    420
   ttggcggcac tggctatcgc ggcgatggcc agcgccagcc tggtgaccgt tgcggtgccc    480
   gcgaccgcca acgccgatcc ggagccagcg cccccggtac ccacaacggc cgcctcgccg    540
   ccgtcgaccg ctgcagcgcc acccgcaccg gcgacacctg ttgccccccc accaccggcc    600
   gccgccaaca cgccgaatgc ccagccgggc gatcccaacg cagcacctcc gccggccgac    660
   ccgaacgcac cgccgccacc tgtcattgcc ccaaacgcac cccaacctgt ccggatcgac    720
   aacccggttg gaggattcag cttcgcgctg cctgctggct gggtggagtc tgacgccgcc    780
   cacttcgact acggttcagc actcctcagc aaaaccaccg gggacccgcc atttcccgga    840
   cagccgccgc cggtggccaa tgacacccgt atcgtgctcg gccggctaga ccaaaagctt    900
   tacgccagcg ccgaagccac cgactccaag gccgcggccc ggttgggctc ggacatgggt    960
   gagttctata tgccctaccc gggcacccgg atcaaccagg aaaccgtctc gctcgacgcc   1020
   aacggggtgt ctggaagcgc gtcgtattac gaagtcaagt tcagcgatcc gagtaagccg   1080
   aacggccaga tctggacggg cgtaatcggc tcgcccgcgg cgaacgcacc ggacgccggg   1140
   ccccctcagc gctggtttgt ggtatggctc gggaccgcca acaacccggt ggacaagggc   1200
   gcggccaagg cgctggccga atcgatccgg cctttggtcg ccccgccgcc ggcgccggca   1260
   ccggctcctg cagagcccgc tccggcgccg gcgccggccg gggaagtcgc tcctaccccg   1320
   acgacaccga caccgcagcg gaccttaccg gccggatc                             1358

## Description

The invention relates to a recombinant Mycobacterium strain expressing a mycobacterial FAP protein under the transcriptional control of a promoter active under hypoxia conditions and its use for the prevention and the treatment of epithelial tumors.

Bladder cancer is among the top five cancers in men and is three to four time less frequent in women. Over 80 % of these cancers are superficial transitional cell carcinomas. *Intravesical immunotherapy with bacillus Calmette-Guerin (BCG) is, today, the reference for treatment and prophylaxis of superficial transitional cell carcinoma of the urinary bladder; it is the most effective therapy for preventing superficial tumor recurrences and treating residual tumors of the bladder after transurethral tumor resection (Lamm et al., N. Engl. J. Med., 1991, 325, 1205-).

While the anti-tumor effect of BCG is well-established, the mechanism through which it occurs is less clear. Some studies suggest that the cell mediated immune response to BCG plays a critical role in the BCG anti-tumor effect. At the same time, other studies identify a direct anti-tumor effect of BCG. The relative contribution of these two possible mechanisms to BCG's clinical anti-tumor activity remains unknown.

The instillation of BCG into the bladder provokes a complex inflammatory response during which lymphocytes and various cytokines are released into the urine. Local granulomas formed in the suburothelial stroma are proposed to play a key role in the control of carcinoma cells *(*Alexandroff et al., The Lancet, 1999, 353, 1689-1694). NK cells have been hypothesized to mediate a part of this control. The production of different cytokines (IL-2, IL-12, IL-18 and interferons α) is over-expressed after BCG therapy and may activate NK cells (Suttmann et al., The Journal of Urology, 2004, 172, 1490-1495). However, the intense immune responses observed in some patients did not correlate with a better disease control and the immunological mechanisms allowing the proliferation control of carcinoma cells are still unclear.

A number of studies demonstrate that BCG exerts a direct anti-proliferative effect on human urothelial carcinoma cells. The alanine and proline rich secreted protein APA (also named FAP-B, antigen MPT-32, 45-kDa glycoprotein, or 45/47-kDa antigen) of the mycobacterial fibronectin attachment protein (FAP) family is a necessary protein for *in vivo* BCG attachment to the bladder epithelial cells and mediation of BCG-induced anti-tumor activity (Zhao et al., Int. J. Cancer, 2000, 86, 83-88). Mycobacterial FAP proteins constitute a family of highly homologous proteins that bind fibronectin in a unique manner. FAP proteins from at least five mycobacterial species including *M. leprae* (FAP-L), *M. avium* (FAP-A), *M. bovis* BCG (FAP-B; GenBank accession number AF013569), *M. smegmatis* (FAP-S) and *M. tuberculosis* (APA, antigen MPT-32, 45-kDa glycoprotein, or 45/47-kDa antigen; EMBL accession number X80268) have been cloned and characterized. The fibronectin binding region (positions 269-292 of FAP-A) contains a conserved RWFV tetrapeptide (positions 273 to 276 of FAP-A) that is necessary for fibronectin binding function. The minimal binding sequence is the 12 amino acid peptide, 269-280 (positions 269 to 280 of FAP-A; Zhao et al., The Journal of Biological Chemistry, 1999, 274, 4521-4526). The APA protein functions as an opsonin, linking BCG to cell surface integrins, via a fibronectin bridge. FAP-mediated BCG adherence to the urothelial carcinoma surface α5β1 integrin, which is the predominant FAP receptor on urothelial cells, induces signaling and gene transactivation pathways involving NF-κB and AP-1. Cell-cycle arrest at the G1/S interface, rather than apoptosis, was reported as being the mechanism contributing to BCG's anti-proliferative effect (Chen et al., BMC Urology, 2005, 5:8).

However, the use of BCG for bladder cancer does not come without drawbacks, both in terms of efficacy and toxicity. First, the response to BCG is unpredictable and not linear. As a result, thirty percent of patients are BCG refractory and there are currently no reliable prognostic factors that accurately predict treatment success or failure. In addition, the long-term durability of response to BCG is limited and the use of maintenance (three weekly treatments every three to six months) or booster therapy (once a month) for up to 36 months is necessary to reduce recurrence of bladder cancer.

Second, BCG has side effects. Most patients experience local symptoms of cystitis including frequency, urgency, dysuria and occasional haematuria. Mild systemic symptoms of high temperature, malaise, and a transient influenza-like illness are also common. Severe side-effects occur in 5 % of patients, roughly 10 % of which involve frank BCG sepsis (Alexandroff *et al.,* precited).

Recent advances in recombinant mycobacteria technology, have open new horizons for bladder cancer immunotherapy. Several approaches have been suggested to produce a recombinant alternative to BCG which is less toxic and/or more effective than the existing BCG (Alexandroff *et al.,* precited):
- a recombinant BCG expressing human cytokines of interest such as interleukin 2, interferon gamma, tumor necrosis factor alpha; it is expected that this recombinant BCG should be more immunopotent and thus could lead to the use of lower doses of BCG and minimize the chance of systemic BCG infection,
- a recombinant strain of BCG which is less virulent or which incorporates a suicide gene or antimicrobial drug susceptibilies that are less toxic and more effectice than conventional antituberculosis drugs; it is expected that these recombinant BCG should produce less side effects.

In addition, it has been suggested to use a non-pathogenic strain of mycobacteria, such as *M*. *smegmatis.*

However, to date, none of these approaches has provided an alternative to BCG immunotherapy. It is in this context that the invention was made.

Knowing that the P_{O2} in urine is low (Leonhardt et al., New Engl. J. Med., 1963, 269, 115-121), i.e. the local P_{O2} of the surface of the bladder epithelium where carcinoma cells are growing, the inventors have engineered a recombinant BCG (BCG Apa⁺⁺) overexpressing the APA protein under hypoxic conditions. In this construct, expression of the APA open reading frame is driven by the mycobacterial α-crystallin promoter. The α-crystallin is a protein which is specifically synthesized during *M. tuberculosis* late exponential and stationary phase growth *in vitro,* following a shift to oxygen-limiting conditions, and may play a role in long-term survival of *M*. *tuberculosis, in vivo* (Yuan et al., J. Bacteriol., 1996, 178, 4484-4492). In *vivo* administration of BCG Apa⁺⁺, leads to overexpression of the APA protein in tissues were the partial pressure of oxygen is low, such as the bladder (Leonhardt et al., N. England J. Medecine, 1963, 269, 115-121). As a result, BCG Apa⁺⁺ is more active than wild-type BCG to control human bladder carcinoma cells proliferation, both *in vitro* and *in vivo.* In addition, BCG Apa⁺⁺ is a potent activator of apoptosis pathways. This recombinant BCG should lower the doses of BCG used and minimize the side-effects associated with BCG immunotherapy.

Therefore, the invention relates to a recombinant vector comprising a mycobacterial FAP protein coding sequence under the transcriptional control of a promoter sequence that is active under hypoxia conditions.

### Definitions

- by "vector" is intended a nucleic acid molecule capable of transporting another nucleic acid to which it has been linked, into a host cell.
- by "expression vector" is intended a vector capable of directing the expression of a cloned polynucleotide.
- by "promoter sequence" is intended a nucleic acid sequence that is necessary to permit an RNA polymerase to initiate transcription of a polynucleotide. Said promoter may also include regulatory sequences (enhancer, repressor), that serve to enhance or repress transcription.
- by "transcriptional control" is intended that the polynucleotide encoding the mycobacterial FAP protein is operatively linked to the promoter (*i.e*. at a position allowing transcription of said polynucleotide and translation of the corresponding protein to occur in the host cells modified by the recombinant expression vector.
- by "hypoxia" is intended a partial pressure of oxygen that is inferior to 30 mm Hg, as determined by conventional techniques, for example by using a polarographic electrode.
- by "mycobacterial FAP protein" is intended a protein encoded by the *apa* gene of *M. tuberculosis* (EMBL accession number X80268; SEQ ID NO: 11; APA CDS from positions 1082 to 2059), or its homolog from other mycobacteria species, including for example the FAP-B gene of *M. bovis* (GenBank AF013569). The *apa* gene of *M*. *tuberculosis* is also named modD, mpt 32 or Rv1860 gene. The APA protein amino acid sequence corresponds to the SwissProt accession number Q50906 (SEQ ID NO: 12).
- by "*acr* gene" or "hspX gene" is intended the gene encoding the alpha-crystallin heat shock protein homolog HSPX (14 kDa antigen; HSP16.3). By reference to *M. tuberculosis* H37Rv sequence (accession number NC_000962.2), the hspX gene (Gene ID: 887579; locus tag Rv2031c) corresponds to positions 2278498 to 2278932 on the DNA minus strand. The hspX gene of *M*. *tuberculosis* corresponds also to GenBank accession number S79751. By reference to *M. bovis* AF2122/97 sequence (accession number NC_002945), the hspX gene (Gene ID: 1094114; locus tag Mb2057c) corresponds to positions 2262845 to 2262411 on the DNA minus strand and the promoter region (SEQ ID NO: 13) that can be amplified with the pair of primers SEQ ID NO: 3 and SEQ ID NO: 4 is included between positions 2263059 and 2262846 on the DNA minus strand. By reference to *M*. *bovis* BCG Strain Pasteur 1173P2 (accession number NC_008769), the hspX gene (Gene ID: 4696845; locus tag BCG_2050c) corresponds to positions 2262150 to 2262584 on the DNA minus strand. Promoter region of hspX includes promoter region of every hspX mycobacterial homolog gene that can be amplified wih the pair of primers SEQ ID NO:3 and SEQ ID NO:4.
- by "FAP coding sequence" is intended the FAP gene, the FAP protein coding sequence (CDS), corresponding to the full-length FAP open reading frame (ORF), or a truncated CDS corresponding to a functional FAP protein. For example, a sequence wherein the 5' end of the FAP open reading frame corresponding to the N-terminal signal sequence of the FAP protein has been deleted.
- by "functional FAP protein" is intended a protein able to bind fibronectin, to inhibit cell proliferation and to induce apoptosis. A functional FAP protein may be a FAP protein fragment or a FAP protein variant having one or more mutation (insertion, deletion, substitution of one or more amino acids) in the FAP protein sequence.

In a first embodiment, the invention features a recombinant vector, wherein said mycobacterial FAP protein is *Mycobacterium tuberculosis* FAP protein (*M. tuberculosis* APA protein).

In a second embodiment, the invention features a recombinant vector, wherein the promoter is a mycobacterial promoter which is active in hypoxic conditions. Examples of such promoters are described in Florczyk et al., Infect. Immun., 2001, 69, 5777-5785 and Florczyk et al., Infect. Immun., 2003, 71, 5332-5343. Among said promoters, the one specified here above may advantageously be used (see chapter definitions, hspX gene); another promoter may also be advantageously used: it corresponds to the promoter of the TB31.7 gene. The promoter sequence may be amplified with the pair of primers SEQ ID NO: 5 and SEQ ID NO: 6, derived from those described in Table 1 of Florczyk et al., (Infect. Immun., 2003, precited), by addition of respectively a Bam*H*I and a *Nco*I restriction site, at the 5'end.

The TB31.7 gene of *M. tuberculosis* corresponds to the locus_tag: Rv2623; Gene ID: 887442; gi2104288. By reference to accession number BX842580 *(Mycobacterium tuberculosis* H37Rv complete genome; segment 9/13), the TB31.7/Rv2623 promoter region (SEQ ID NO: 14) is included between positions 176190 and 176560.

By reference to *M. bovis* BCG Strain Pasteur 1173P2 (accession number NC_008769), the TB31.7 gene (locus tag BCG_2650) corresponds to positions 1459592 to 1460485 on the DNA minus strand. Promoter region of the TB31.7 gene includes promoter region of every TB31.7 mycobacterial homolog gene that can be amplified with the pair of primers SEQ ID NO:5 and SEQ ID NO:6.

Preferably, the mycobacterial promoter is the promoter of the *acr* gene (hspX promoter); expression of the alpha-crystallin heat shock protein homolog HSPX (14 kDa antigen; HSP16.3) encoded by the mycobacterial *acr* gene, is specifically induced to very high level under hypoxic conditions.

More preferably, the vector comprises a *M. tuberculosis* APA protein coding sequence under the transcriptional control of the hspX promoter.

A vector according to the present invention comprises, but is not limited to, a YAC (yeast artificial chromosome), a BAC (bacterial artificial), a phage, a phagemid, a cosmid, a viral vector, a plasmid, a RNA vector or a linear or circular DNA or RNA molecule which may consist of chromosomal, non chromosomal, semisynthetic or synthetic nucleic acids. Preferred vectors are those capable of autonomous replication and/or expression of nucleic acids to which they are linked. One type of preferred vector is an episome, i.e., a nucleic acid capable of extra-chromosomal replication. In general, expression vectors of utility in recombinant DNA techniques are often in the form of "plasmids" which refer generally to circular double-stranded DNA loops which, in their vector form are not bound to the chromosome. Large numbers of suitable vectors are known to those of skill in the art.

The vector according to the present invention comprises an expression cassette, wherein the sequence encoding the FAP protein is placed under control of appropriate transcriptional and translational control elements to permit production or synthesis of said protein. More particularly, the vector may comprise additional features which are well-known in the art, including:
- a cloning site located downstream of the promoter region, for insertion of the ORF. The ATG initiation codon and/or the stop codon of the ORF may be included in a restriction site for allowing insertion of the protein coding sequence in frame with the vector. Optionally, the vector may comprise a multiple cloning site, comprising multiple adjacent cloning sites allowing any insert to be cloned in the vector in frame with any reading frame in the vector (fusion proteins). For example, the 5' or 3' end of the ORF can be fused to a sequence encoding a tag. The tag may be used for the purification (polyhistidine sequence) or the detection (B epitope, fluorescent protein) of the expressed protein. Optionally, the 5' or 3' end of the ORF and the sequence encoding the tag are separated with a cleavage site for a protease/endopeptidase sequence to allow removal of the tag.
- at least one selectable marker, for example an auxotrophy marker, an antibiotic resistance gene (tetracycline, rifampicin, ampicillin, kanamycin resistance) or a mercury resistance gene, for the selection of recombinant bacteria.
- a transcription termination signal (terminator), downstream of the ORF; to cause RNA polymerase from the host to terminate transcription of the transcript generated by the promoter.
- at least one origin of replication for the maintenance of the vector in the host.

In a fourth embodiment, the invention features a vector which is a plasmid (double-stranded circular DNA). Preferably, said plasmid contains a bacterial origin of replication and an antibiotic resistance gene. In particular, the invention features a plasmid, identified as pYAPA2031, wherein the *M*. *tuberculosis* APA protein coding sequence under the transcriptional control of the hspX promoter is inserted in the *EcoR*V site of the pYUB295 plasmid (Dussurget et al., Infect. Immun., 2001, 69, 529-533; Gomez et al., Mol. Microbiol., 1998, 29, 617-628).

The invention concerns also an expression cassette derived from the recombinant vector according to the present invention, consisting of a polynucleotide fragment comprising the promoter sequence which is active under hypoxia conditions operatively linked to the mycobacterial FAP protein coding sequence, as defined above. Examples of expression cassettes are SEQ ID NO: 18 and SEQ ID NO: 19 wherein the *M. tuberculosis* APA coding sequence (SEQ ID NO: 15) is under the control of respectively the *M. bovis* AF2122/97 hspX/*acr* promoter (SEQ ID NO: 16) and the *M. tuberculosis* TB31.7/Rv2623 promoter (SEQ ID NO: 17). Said expression cassette may further comprise additional features as defined above (cloning site, tag, downstream termination signal).

The invention relates also to a host cell which is genetically modified by a recombinant vector as defined above (recombinant cell).

Particularly, the invention relates to bacteria, preferably mycobacteria transformed by a recombinant vector as defined above.

Preferred mycobacteria include those of *M. tuberculosis* complex, such as *M. bovis* BCG strains, for example the Pasteur BCG strain. Other preferred mycobacteria are those which are fast growing and non pathogenic for humans such are for example, *Mycobacterium smegmatis, Mycobacterium fortuitum* and *Mycobacterium microti.*

In particular, the invention features a recombinant *Mycobacterium bovis* BCG strain, stably transformed with the plasmid pYAPA2031, deposited at the Collection Nationale de Cultures de Microorganismes, 25 rue du Docteur Roux, 75724 Paris Cedex 15, on July 21 2006, under accession number I-3659, identified hereafter as BCG Apa⁺⁺.

The invention relates also to an antitumoral composition comprising a suitable amount of a recombinant vector or host cell as defined above, in an acceptable carrier, such as a stabilizer, a buffer and the like.

The composition according to the present invention may comprise living or killed vector/cell. The killed vector/cell compositions are prepared by any means, known to those of ordinary skill in the art.

A pharmaceutical composition or formulation refers to a form suitable for administration (oral, topical, by injection or inhalation) into a subject, for example a mammal, and in particular a human. Suitable forms, in part, depend upon the use or the route of entry. A preferred formulation is for topical administration, more preferably for intravesical, intravaginal, rectal administration, or for inhalation.

A pharmaceutically effective dose is that dose required to prevent, inhibit the occurrence or treat (alleviate a symptom to some extent, preferably all the symptoms) of a disease or state. The pharmaceutically effective dose of the vector/host cell depends upon the composition used, the route of administration, the type of mammal being treated, the physical characteristics of the specific mammal under consideration, concurrent medication, and other factors, that those skilled in the medical arts will recognize. Generally, the effective dose of recombinant mycobacteria for human, is between 10⁶ and 10¹⁰CFU (Colony Forming Unit) or CFU equivalent.

Preferably, the tumor is an epithelial tumor. More preferably, the tumor is a superficial tumor. In particular, the tumor is a transitional cell carcinoma of the bladder or a lung, colon or cervix carcinoma.

In a first embodiment, the antitumoral composition according to the present invention, comprises a recombinant mycobacteria strain as defined above, in particular the BCG Apa⁺⁺ strain.

Preferably, the recombinant mycobacteria strain is killed, more preferably by extended freeze-drying. The method of preparation of extended-freeze-drying mycobacteria strains is described in the PCT International Application WO 03049752.

In a second embodiment, the antitumoral composition according to the present invention, further comprises an immunostimulatory agent.

The invention relates also to the use of a recombinant vector or host cell as defined above for the manufacture of a medicament intended for the prevention or the treatment of a tumor in a subject.

The invention relates also to a product containing a recombinant vector or a host cell as defined above, and an immunostimulatory agent, as combined preparation for simultaneous, separate or sequential use in antitumoral therapy.

The invention relates also to a method for preventing or treating a tumor in a subject, comprising: administering to the subject, an effective amount of a composition, as defined above, by any appropriate means. Particularly, the composition is administered intravesically, intravaginally, rectally, or by inhalation and comprises between 10⁶ and 10¹⁰ CFU (Colony Forming Unit) or CFU equivalent of recombinant mycobacteria according to the invention.

The invention relates also to the use of the expression cassette or recombinant vector, as defined above for the preparation of a modified host cell, preferably a recombinant mycobacteria strain wherein the expression cassette is integrated in the genome of the recombinant mycobacteria.

The invention also relates to a host cell which is genetically modified and has the expression cassette as defined above, integrated in its genome.

The practice of the present invention will employ, unless otherwise indicated, conventional methods of microbiology, molecular biology and immuno-biology within the skill of the art. Such techniques are explained fully in the literature.

The recombinant vector according to the invention is constructed and introduced in a host cell by the well-known recombinant DNA and genetic engineering techniques using classical methods, according to standard procedures as those described in: Current Protocols in Molecular Biology (Frederick M. AUSUBEL, 2000, Wiley and son Inc, Library of Congress, USA*)* and Molecular Cloning: A Laboratory Manual, Third Edition, (Sambrook et al, 2001, Cold Spring Harbor, New York: Cold Spring Harbor Laboratory Press*).*

In addition to the preceding features, the invention further comprises other features which will emerge from the description which follows, which refers to examples illustrating the BCG recombinant strain and its use according to the invention, as well as to the appended drawings in which:

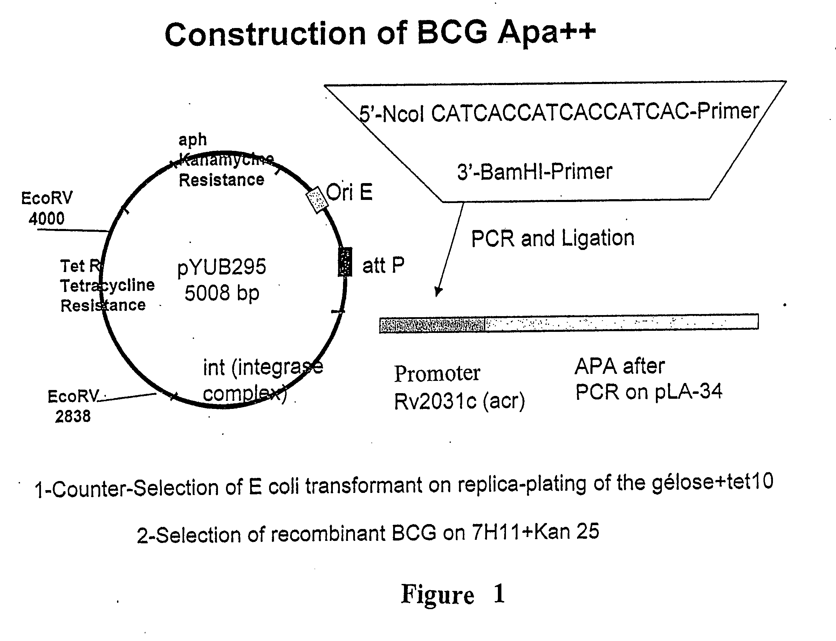

- Figure 1 illustrates the construction of the recombinant strain BCG Apa⁺⁺ containing the APA coding sequence under the transcriptional control of the hspX promoter (derived from the pYAPA2031 plasmid), integrated into the BCG genome.

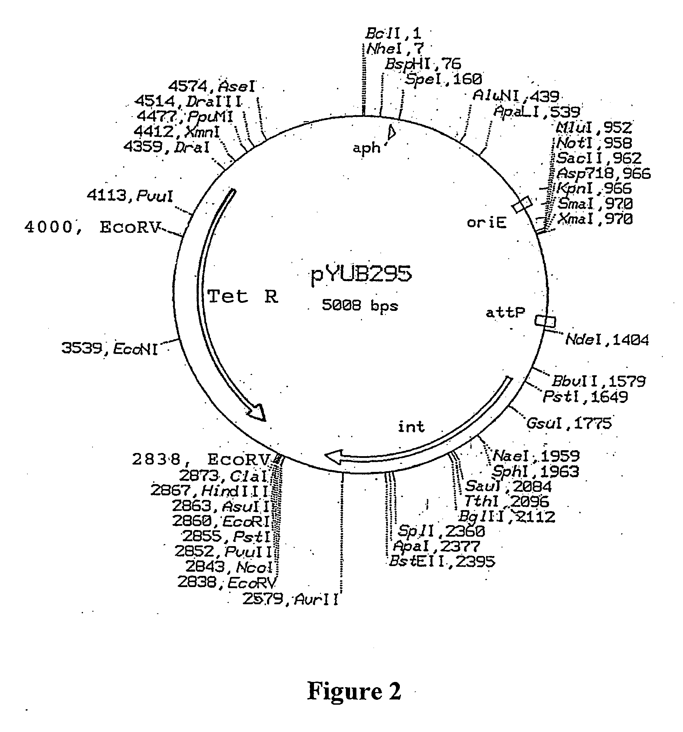

- Figure 2 is the map of the plasmid pYUB295.

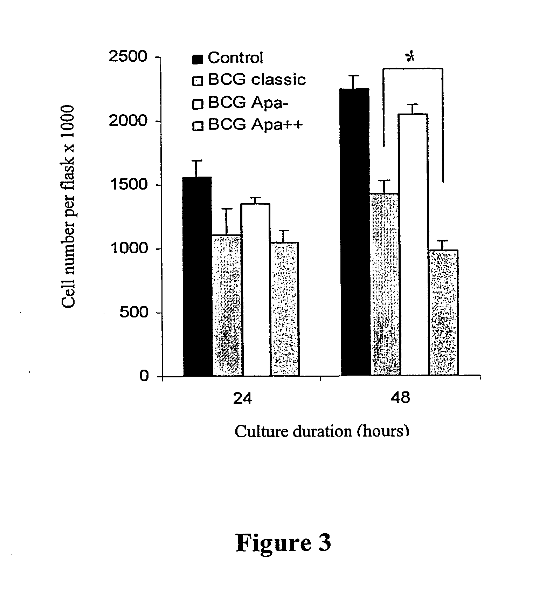

- Figure 3 illustrates inhibition of T24 human bladder carcinoma cell growth by BCG or BCG Apa⁺⁺. Cells were cultivated 24 h or 48 h in the presence of 5.10⁶ BCG (BCG classic), BCG Apa⁺⁺ or BCG Apa⁻ bacilli, or without bacteria (Control). Cells were harvested and live cells were counted by trypan blue exclusion assay. Data reported as mean ± s.e.m (n=5).

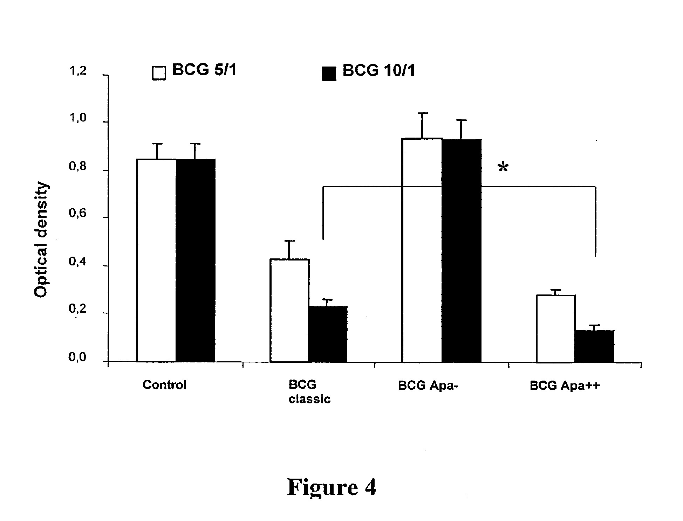

- Figure 4 illustrates inhibition of T24 human bladder carcinoma cell viability by BCG or BCG Apa⁺⁺. Cells were cultivated 48 h in the presence of, either 5.10⁶ or 10⁷ BCG (BCG classic), BCG Apa⁺⁺ or BCG Apa⁻ bacilli (equivalent to 5 (BCG 5/1) and 10 (10/1) bacilli per cell, respectively) or without bacteria (Control). Cells were harvested and viability was evaluated by MTT assay. Data are reported as mean ± s.e.m (n=5).

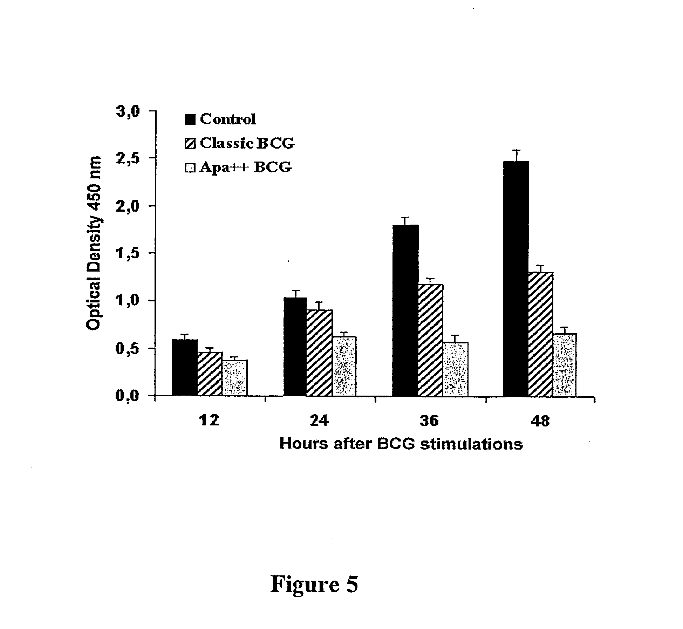

- Figure 5 illustrates inhibition of T24 human bladder carcinoma cell proliferation by BCG or BCG Apa⁺⁺. Cells were cultivated for different time (12 h, 24 h, 36 h or 48 h) in the presence of 5.10⁵ BCG or BCG Apa++ bacilli (10 bacilli/cell), or without bacteria (Control) and DNA synthesis was evaluated by BrdU incorporation assay. Data are reported as mean ± s.e.m (n=5).

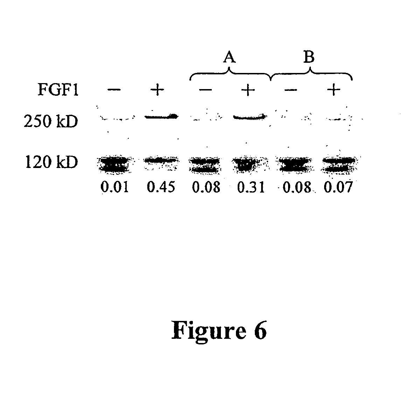

- Figure 6 illustrates inhibition of FGR-3 dimerisation by BCG or BCG Apa⁺⁺ in T24 human bladder carcinoma cells treated with FGF1. Cells were incubated 48 h with 10⁶ CFU/ml (Colony Forming Unit per milliter) of BCG (A) or BCG Apa⁺⁺(B) bacilli or without bacteria, cooled at 4°C during 15 min, incubated for 2 h at 4°C with (+) or without (-) FGF-1 (50 ng/ml). After covalent cross-linking with bis-(sulfosuccinimidyl)-suberate and disuccinimidyl-suberate, the cells were lysed, immunoprecipitated with anti-FGFR-3 C-terminal antibody and probed on an immunoblot with a polyclonal antibody to the cytoplasmic domain of FGFR-3. The values represent the density obtained for the dimers (∼ 250 kDa) relative to the sum of the density ofmonomers (∼120 kDa) and dimers.

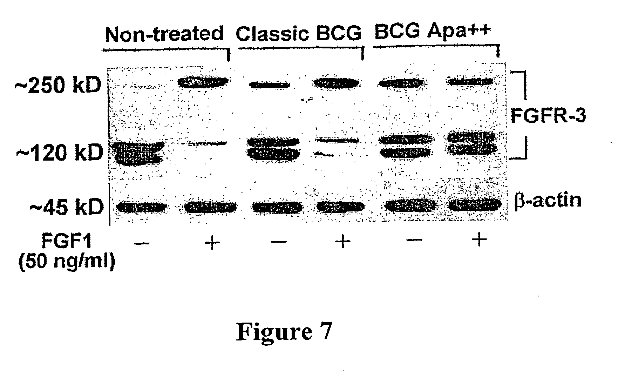

- Figure 7 illustrates inhibition of FGR-3 dimerisation by BCG or BCG Apa⁺⁺ in RT112 human bladder carcinoma cells treated with FGF1. Cells were incubated 48 h with 10⁶ CFU/ml of BCG ("Classic BCG") or BCG Apa⁺⁺ or without bacteria (non-treated), cooled at 4°C during 15 min, and incubated for 2 h at 4°C with (+) or without (-) FGF-1 (50 ng/ml). After covalent cross-linking with bis-(sulfo-succinimidyl)-suberate and disuccinimidyl-suberate, the cells were lysed, immunoprecipitated with anti-FGFR-3 C-terminal antibody and probed on an immunoblot with a polyclonal antibody to the cytoplasmic domain of FGFR-3. The values represent the density obtained for the dimers (∼ 250 kDa) relative to the sum of the density of monomers (∼120 kDa) and dimers.

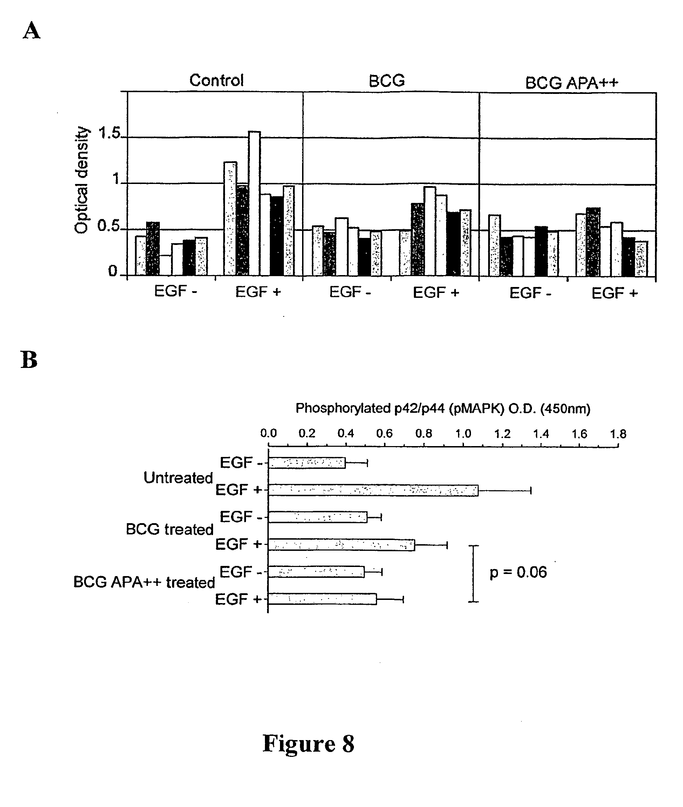

- Figure 8 illustrates stimulation of MAPK pathway by BCG or BCG Apa⁺⁺ in T24 human bladder carcinoma cells treated with EGF. Cells were incubated, 48 h with 10⁶ CFU/ml of BCG or BCG Apa⁺⁺ or without bacteria (Control/untreated), then 15 minutes with (+) or without (-) EGF (100 ng/ml) and lysed. The presence of phosphorylated mitogen-activated protein kinase p42/p44 (pMAPK) was determined by ELISA. A. Data reported well by well. **B**. Data reported as mean ± s.e.m.

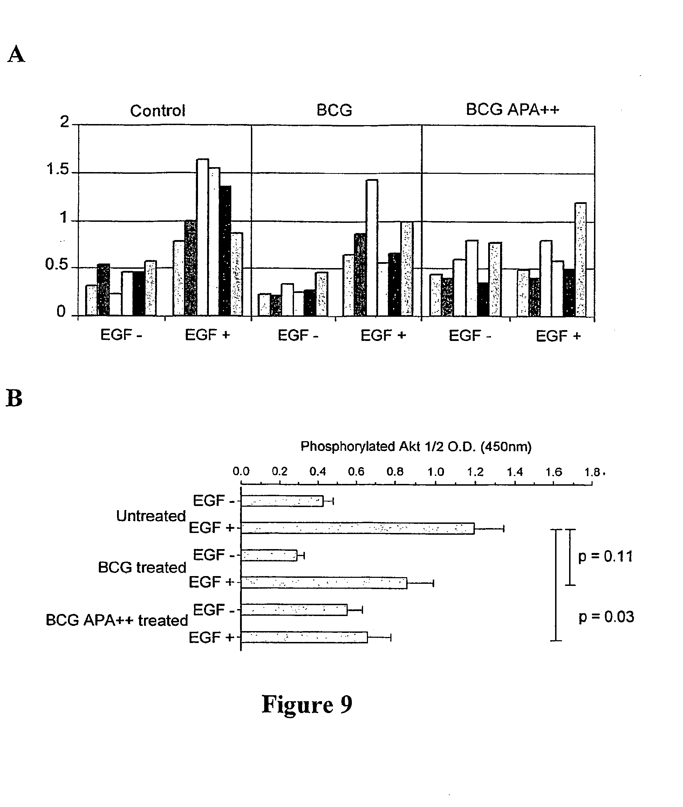

- Figure 9 illustrates stimulation of Akt1-2 pathway by BCG or BCG Apa⁺⁺ in T24 human bladder carcinoma cells treated with EGF. Cells were incubated, 48 h with 10⁶ CFU/ml of BCG or BCG Apa⁺⁺ bacilli or without bacteria, then 15 minutes with (+) or without (-) EGF (100 ng/ml) and lysed. The presence of phosphorylated Akt 1-2 was determined by ELISA. **A**. Data reported well by well. **B**. Data reported as mean ± s.e.m.

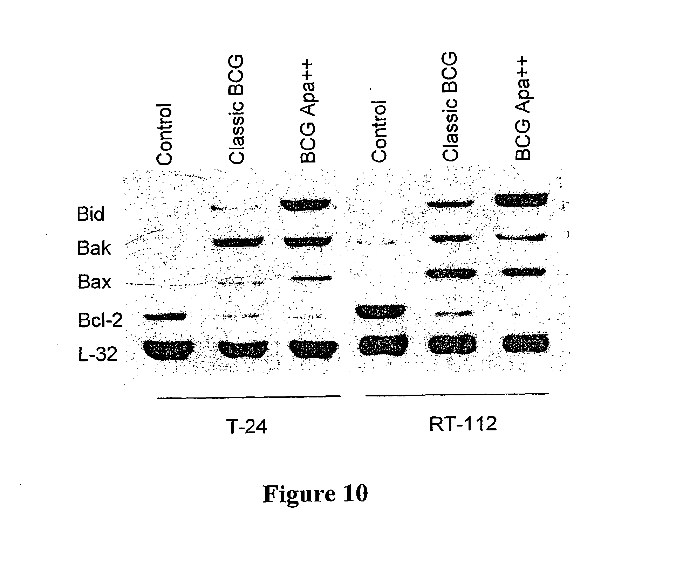

- Figure 10 illustrates the induction of apoptotic pathways by BCG or BCG Apa⁺⁺ in T24 and RT112 human bladder carcinoma cells, evaluated by Bcl-2, Bid, Bak and Bax mRNA expression levels. Cells were incubated 24 h with 5.10⁶ CFU of BCG or BCG Apa⁺⁺ or without bacteria (control). mRNA was extracted and Bc1-2, Bid, Bak and Bax mRNA expression levels were analysed by RNAse protection assay.

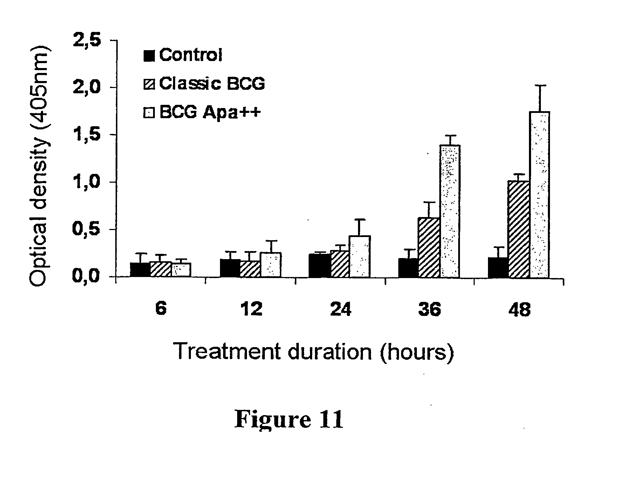

- Figure 11 illustrates the induction of apoptotic pathways by BCG or BCG Apa⁺⁺ in T24 human bladder carcinoma cells, evaluated by caspase-8 activity. T24 cells were incubated 6 hours, 12 hours, 24 hours, 36 hours or 48 hours with BCG or BCG Apa++ or without bacteria (Control). Caspase-8 was measured on cell lysates using colorimetric assay. Ribosomal protein L-32 mRNA was used as control. Data expressed as mean ± s.e.m for n=8. The difference between BCG and BCG Apa⁺⁺ were significant (p < 0.05) after 36 h or 48 h.

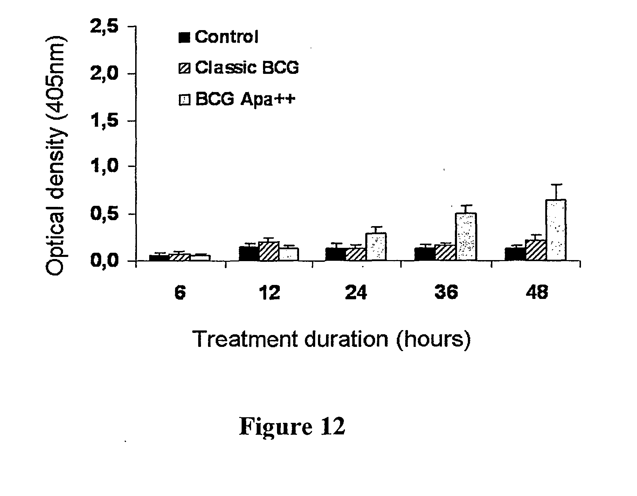

- Figure 12 illustrates the induction of apoptotic pathways by BCG or BCG Apa⁺⁺ in T24 human bladder carcinoma cells, evaluated by caspase-9 activity. analysis. T24 cells were incubated 6 hours, 12 hours, 24 hours, 36 hours or 48 hours with BCG or BCG Apa++ or without bacteria (Control). Caspase-9 was measured on cell lysates using colorimetric assay. Ribosomal protein L-32 mRNA was used as control. Data expressed as mean ± s.e.m for n=8. The difference between BCG and BCG Apa⁺⁺ were significant (p < 0.05) after 36 h or 48 h.

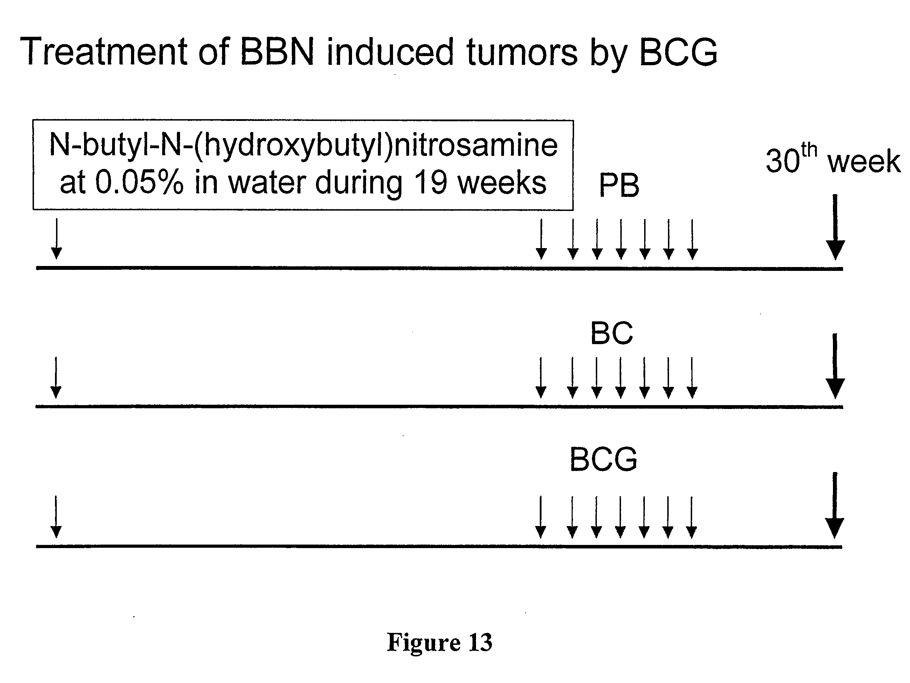

- Figure 13 illustrates the protocol of BBN induced bladder-tumour generation and subsequent tumour treatment by BCG. Rats were given 0.05% N-butyl-N-(4-hydroxybutyl) nitrosamine (BBN) at 0.05% in drinking water for 19 weeks. One week after the end of BBN treatment (20^{th} week), the rats received 6 weekly intravesical instillations (0.5 ml) of PBS alone, or PBS containing 10⁸ CFU/ml of BCG or BCG Apa⁺⁺.

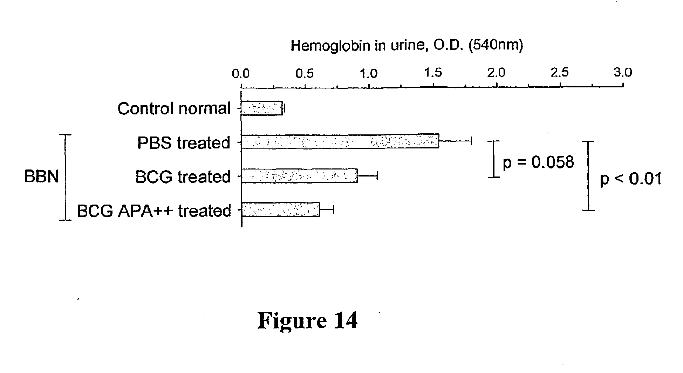

- Figure 14 illustrates inhibition of hematuria by BCG. Rats having developed bladder tumours following a 19 week BBN administration in drinking water, received (week 20) 6 weekly intravesical instillations (0.5 ml) of PBS alone (PBS treated), or PBS containing 10⁸ CFU/ml of BCG (BCG treated) or BCG Apa⁺⁺. (BCG APA⁺⁺ treated). Rats receiving normal drinking water for 19 weeks and maintained untreated for 30 weeks were used as normal controls. The urines were harvested at week 30 and their content in haemoglobin measured using a colorimetric assay.

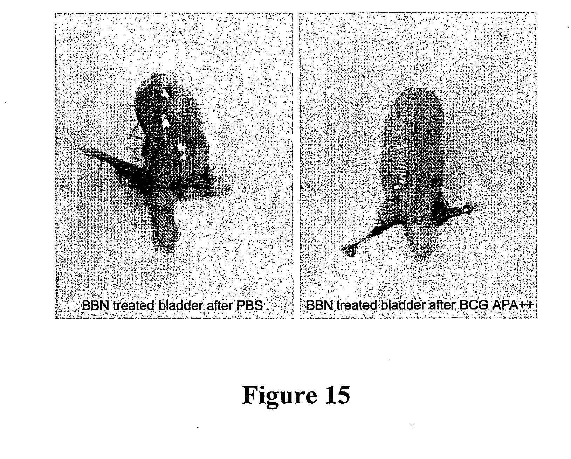

- Figure 15 illustrates the antitumoral effect of BCG Apa⁺⁺ *in vivo.* Rats having developed bladder tumours following a 19 week BBN administration in drinking water, received (week 20) 6 weekly intravesical instillations (0.5 ml) of PBS alone or PBS containing 10⁸ CFU/ml of BCG Apa⁺⁺ (BCG APA⁺⁺). Rats were euthanized at the 30^{th} week (one month after the last BCG treatment), bladders were collected and subjected to clinical examination (macroscopical observation). Left panel: bladder collected from rat receiving BBN and treated with PBS. Right panel: bladder collected from rat receiving BBN and treated with BCG Apa⁺⁺.

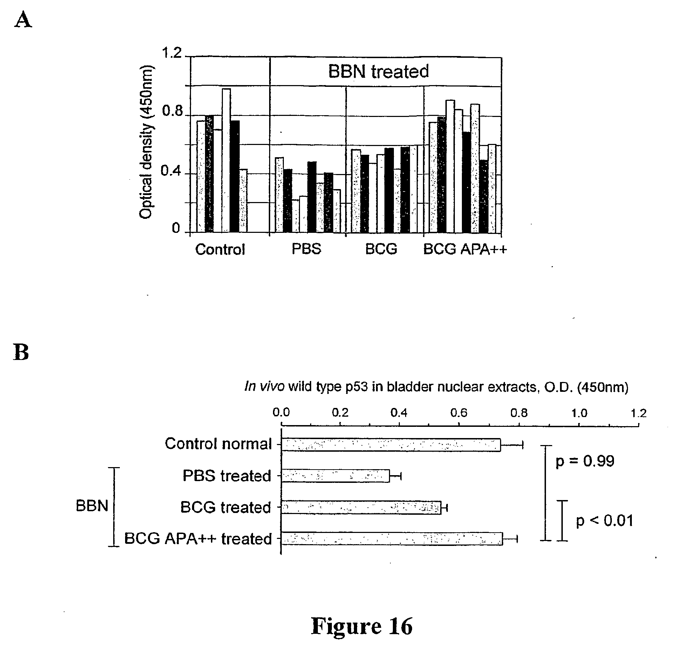

- Figure 16 illustrates the activation of wild type p53 in nucleus of cells of BBN-induced bladder tumors from rats which received 6 weekly intravesical instillations (0.5 ml) of PBS alone, or PBS containing 10⁸ CFU/ml of BCG vaccine or BCG Apa⁺⁺. Rats were euthanized at the 30^{th} week (one month after the last BCG treatment) and activated wild type p53 were measured by an Enzyme Immunometric Assay. A. Data reported well by well. **B.** Data reported as mean ± s.e.m.

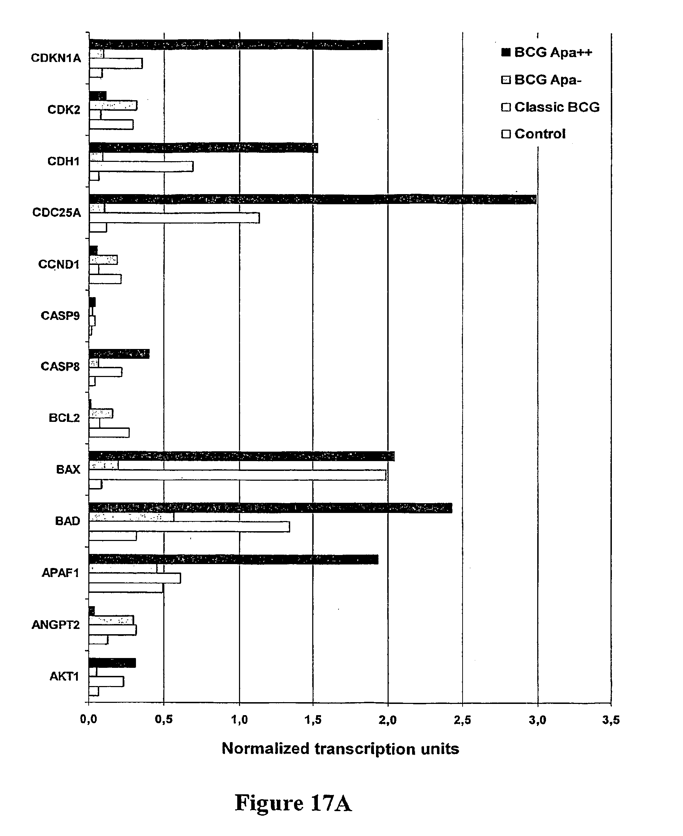

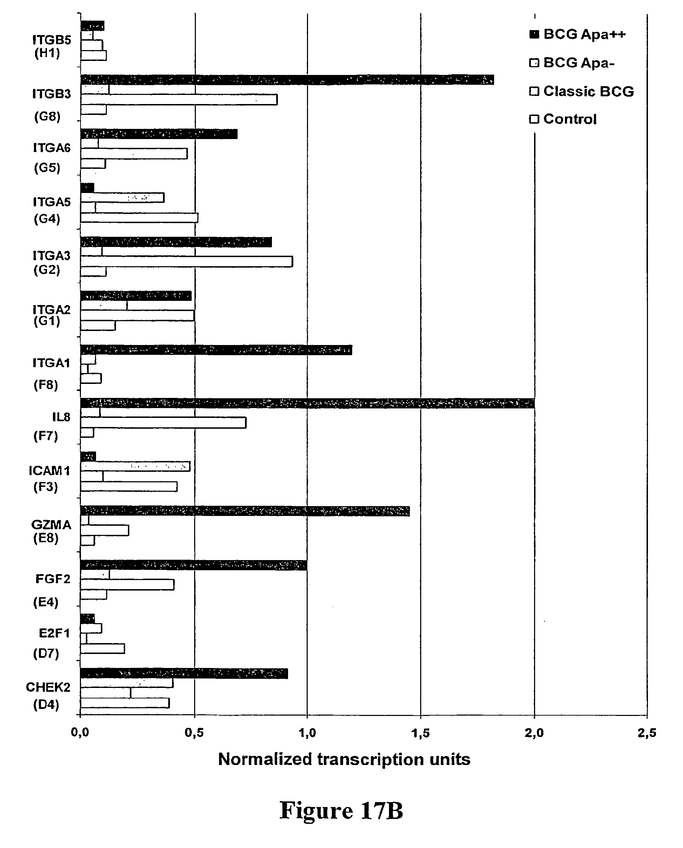

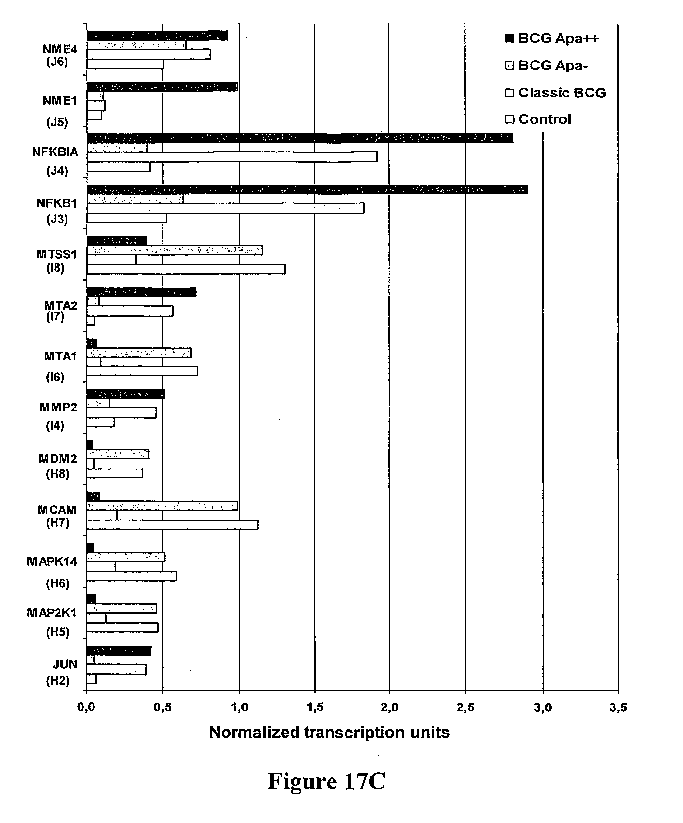

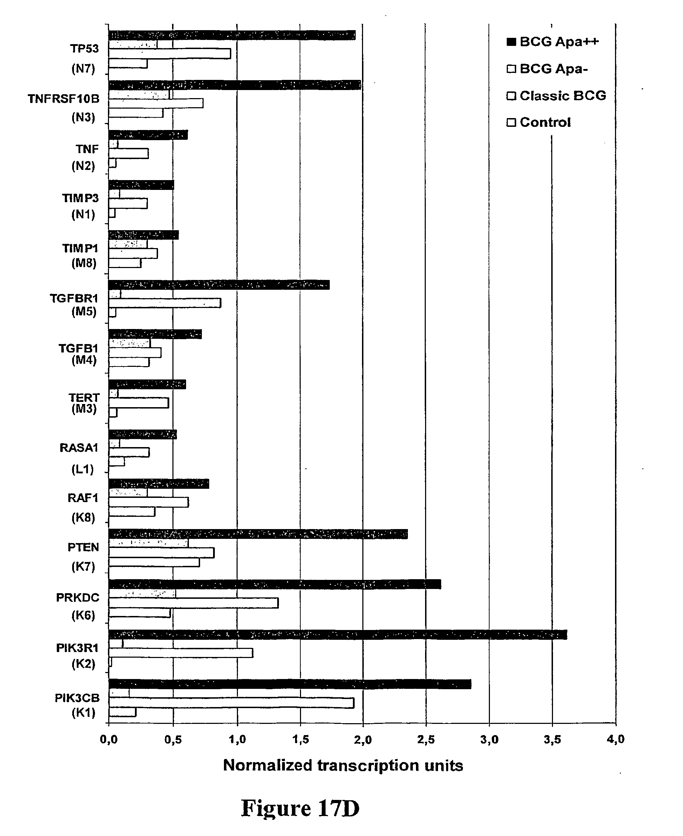

- Figures 17 A, B, C and D illustrate the transcriptome of T24 cells treated with BCG, BCG Papa++, BCG Apa-, or without bacteria analysed by microarray using specific cDNA fragments of 113 genes associated with different cancer cascades and classified in six groups (Table II). The figures were drawn from the quantitative data presented in Table I. Data are reported as mean ± s.e.m (n=3). Transcription units were normalized as indicated in example 6.

### Example 1: Construction of a recombinant BCG (BCG Apa⁺⁺) overexpressing the APA protein under the control of the alpha-crystallin promoter.

To construct a recombinant BCG able to produce APA molecules when the bacteria are under hypoxia, a plasmid containing the appropriate sequences was tailored. This plasmid contains the *apa* gene of *M*. *tuberculosis* (EMBL accession number X80268 and Laqueyrie et al., Infection and Immunity, 1995, 63, 4003-4010) coding for the APA protein, cloned under the transcriptional control of the hspX (GenBank accession number S79751; Yuan et al., J. Bacteriol., 1996, 178, 4484-4492; Florczyk et al., Infect. Immun., 2001, 69, 5777-5785 and Florczyk et al., Infect. Immun., 2003, 71, 5332-5343), and the *aph* gene (kanamycin resistance). Bacteria of the wild-type BCG 1173P2 Pasteur strain, were then electroporated with the plasmid, and recombinant BCG overexpressing APA (BCG Apa⁺⁺) was isolated in Sauton medium containing kanamycin (Figure 1).

The *M. tuberculosis* APA coding sequence was amplified by PCR from pLA34-2 (Laqueyrerie et al., Infect. Immun. 1995, 63, 4003-4010). The *apa* sequence was modified to create a *Nco*I site via the forward primer (5'-catgccatggtacaggtggaccccaacttgaca-3': SEQ ID NO: 1) and a *Bam*HI site in the reverse sequence (5'-ttaggatccggccggtaaggtccgctgcggtgt-3': SEQ ID NO: 2) in order to subclone the *Nco*I*-Bam*HI PCR product into the pQE60 expression vector (QIAGEN; (Horn et al., J. Biol. Chem., 1999, 274, 32023-32030).

Two inducible BCG promoters with *Bam*HI and *Nco*I cohesive ends were generated by PCR performed on Pasteur *M*. *bovis* BCG DNA, using primers BamhspX R (5'-ttaggatccgtccggcatgatcaacctcc-3': SEQ ID NO: 3) and NcohspX F (5'-catgccatggggtggccatttgatgcctcc-3': SEQ ID NO: 4) for alpha-crystalline (*acr*) promoter and the primers BamRv2623 F (5'-ttaggatccgggccatggactggtcgtcg-3': SEQ ID NO: 5) and NcoRV2623 R (5'-catgccatggacatcgcggtcctcctgtcg 3': SEQ ID NO: 6) for Rv2623 promoter (Florczyk *et al.,* 2001 and 2003, precited).

These fragments were T4 ligated and after two Klenow treatments, *acr*-apa or Rv2623-apa cassettes were inserted into *EcoR*V restricted plasmid pYUB295 (Dussurget et al.,.Infect Immun., 2001, 69, 529-533; Gomez et al.,.Mol. Microbiol. 1998, 29, 617-628;Figure 2). This insertion created approximately 5.5 kbp plasmids of pYAPA2031 and pYAPA2623. After amplification in *E*. *coli* (XL-1 Blue) and tetracycline sensitivity replica plating, sensitive clones were selected. The plasmids were purified and electroporated into BCG. They do not replicate in BCG but contains the attachment site (attP) and the integrase gene (int) of the mycobacteriophage L5 that direct the integration of the plasmid at the attB site of the mycobacterial chromosome with high efficiency (Lee et al., Proc Natl Acad Sci USA, 1991, 29, 3111-3115).

In particular, the invention features a recombinant *Mycobacterium bovis* BCG strain, stably transformed with the plasmid pYAPA2031, deposited at the Collection Nationale de Cultures de Microorganismes, 25 rue du Docteur Roux, 75724 Paris Cedex 15, on July 21 2006, under accession number I-3659, identified hereafter as BCG Apa⁺⁺.

Low-oxygen liquid cultures were grown in vented-cap tissue culture flasks (25 cm² Coming) in an incubator which allowed to control the oxygen tension. Vented-cap tissue culture flasks allowed for the exchange of gases between the flasks and the controlled environment of the incubator. Oxygen levels were maintained by injecting prepared gas mixture (Carboxique) into the incubators. Low-oxygen cultures were grown under atmospheres of 1.3 % total O₂. High-oxygen cultures were grown in ambient air in 25 cm² tissue culture flasks with the caps tightly sealed. When necessary, kanamycin was used at 25 µg/ml.

Overexpression was confirmed by Western blotting using a rabbit polyclonal antibody raised against the *M. bovis BCG* APA (Laqueyrerie *et al.,* Horn *et al*., precited).

### Example 2: Construction of a BCG apa⁻ mutant (BCG Apa)

The BCG *apa* gene was mutated by allelic exchange (Pelicic et al., Proc. Natl. Sci. USA, 1997, 94, 10955-10960). Two DNA fragments containing 1000 bp of *apa* flanking sequences were generated by PCR using primers Sma15up (5-tcccccgggggtgttgacccgacac-3; SEQ ID NO: 7)) and Pst23up (5-aactgcagggcgaagaacctacc-3; SEQ ID NO: 8) for upstream fragment (U) and Pst35down (5-aactgcagccaagtgatacccct-3; SEQ ID NO: 9) and Hin43down (5-cccaagcttggagatcggtgcggc-3; SEQ ID NO: 10) for downstream fragment (D). These fragments were cloned into pBluescript II KS (INVITROGEN), constructing pUD1. pApa::Kan was created by cloning the HincII-flanked *aph* gene (kanamycin resistance) of pUC4K into the Apa *Kpn*I site of pLA34-2 (Horn et al., J. Biol. Chem., 1999, 274, 32023-32030). A 3.3 kb blunt ended fragment of pApa::Kan containing *A*pa::*aph* was cloned into the *PstI* site of pUD1 creating pUDApa::Kan whose *Sma*I-HindIII fragment was subcloned in *Sma*I site of pXYL4, a plasmid bearing the *xylE* gene (Pelicic *et al.,* precited). The 6-kb *Bam*HI fragment containing U, D, Apa::*aph* and *xylE* was isolated and ligated at the *Bam*HI site of pPR27, a vector which contains the counterselectable *sacB* gene and the thermosensitive origin of replication of pAL5000 (Pelicic *et al.,* precited). The resulting plasmid pPR27::Xyl::UDApaKan was electroporated into *BCG* and transformants were selected at 32°C on 7H11 medium containing kanamycin (25 µg/ml) and then grown in 7H9 broth containing kanamycin. Gene replacement accompanied by plasmid loss was selected on 7H11-kanamycin-2% sucrose at 39°C (Pelicic *et al.,* precited). Loss of the plasmid was confirmed in 100% of the resultant colonies by spraying with catechol, a chromogenic substrate of XylE *(Pelicic et al.,* precited). Gene replacement *of apa* was verified by PCR of genomic DNA from three colonies, using the primers *of apa* gene (Horn *et al.,* precited) and the primers for *aph* gene *(Norman, E., Gene replacement in Mycobacterium bovis BCG. Methods in Molecular Biology Vol: 101, Mycobacteria Protocols Edited by T. Parish and N.G. Stoker. Humana Press 1998).* One mutant clone was designated BCG Apa⁻. The absence of APA from the mutants was confirmed by Western blotting using a rabbit polyclonal antibody raised against the *M. bovis BCG Apa* (Laqueyrerie, Infect Immun., 1995, 10, 4003-4010; Horn et al., precited).

### Example 3: Inhibition of human carcinoma cell growth in vitro by BCG Apa⁺⁺ or BCG

*In vitro* studies were performed in two cell lines established from human bladder carcinomas: T24 cells, derived from a human transitional cell carcinoma (ATCC # HTB-4) and RT112 or RT112/84 cells (ECACC 85061106; Marshall C. et al., J. Natl. Cancer Inst., 1977, 58, 6, 1743-1751), derived from a human papillary non-metastatic bladder carcinoma using standard cell culture protocols, which are well-known to those skilled in the art.

T24 and RT112 cells were cultivated at 37°C, in a humid atmosphere with 5% CO₂, in DMEM (GIBCO) cell culture medium supplemented with 10% decomplemented fetal bovine serum (FBS, EUROBIO) and 1 % non-essential amino acids.

In the following studies, *Mycobacterium bovis* BCG 1173P2 (The Pasteur strain) will be named BCG or classic BCG, recombinant BCG Apa⁺⁺ will be named BCG Apa⁺⁺ and the mutant BCG Apa⁻, BCG Apa⁻.

### 1) Inhibition of cell growth

T24 cells (10⁶) were seeded in 25 cm² culture flasks and cultivated in 10 ml culture medium. After 48 hours of culture, 5.10⁶ BCG bacilli (BCG, BCG Apa⁻ or BCG Apa⁺⁺) were added to cell culture medium. After 24 or 48 hours of culture in the presence of BCG, the cells were harvested and living cells, excluding trypan blue, were counted in a Malassez cell.

Figure 3 shows that BCG treatment inhibits carcinoma cell growth. The effect which is much higher with BCG Apa⁺⁺ is abolished when the *apa* gene is inactivated (BCG Apa⁻), indicating that bacterial corpse are not directly implicated and moreover that APA molecules are essential to inhibit cell growth.

### 2) Inhibition of cell viability

The effect of BCG and BCG Apa⁺⁺ on cell viability was evaluated by MTT assay, using Cell Proliferation Kit I (MTT) from ROCHE DIAGNOSTICS. The MTT assay is based on the cleavage of the yellow tetrazolium salt MTT (3-[4,5-dimethylthiazol-2-yl]-2,5-diphenyl tetrazolium bromid) to purple formazan crystals by metabolic active cells. This cellular reduction involves the pyridine nucleotide cofactors NADH and NADPH. The formazan crystals formed are solubilized and the resulting colored solution is quantified using a scanning multiwell spectrophotometer.

T24 cells were cultivated for 48 hours in the presence of 5.10⁶ or 10⁷ BCG, BCG Apa⁻ or BCG Apa⁺⁺ bacilli (equivalent to 5 and 10 bacilli per cell, respectively). At the end of the incubation period, the cells were transferred into microplates (tissue culture grade, 96 wells, flat bottom) in a final volume of 100 µl culture medium per well. Twenty four hours after cell transfer, 10 µl of the MTT labeling reagent (0.5 mg/ml) was added to each well. After an incubation period of 4h, at 37°C in 5% CO2, 100 µl of the solubilization solution was added into the wells. The plates were incubated overnight at 37°C and the reaction products were quantified by measuring the absorbance at 570 nm using a scanning multiwell spectrophotometer.

Figure 4 shows that cell viability is reduced after BCG or BCG Apa⁺⁺ treatment. The effect is abolished when the *apa* gene is inactivated (BCG Apa⁻), indicating that bacterial corpse are not directly implicated and moreover that APA molecules are essential to reduce cell viability.

### 3) Inhibition of cell proliferation

The effect of BCG and BCG Apa⁺⁺ on cell proliferation was evaluated by BrdU incorporation assay, using Cell Proliferation ELISA BrdU (colorimetric) kit (ROCHE DIAGNOSTICS).

T24 cells (2.5x10⁴/well) were grown in presence of 2.5x10⁵ BCG or BCG Apa++ bacilli (10 bacilli/cell) for 12 hours, 24 hours, 36 hours or 48 hours at 37°C. At the end of each measuring time, 10 µM BrdU was added to the cells for 3 hours. During this labelling period, the pyrimidine analogue BrdU was incorporated in place of thymidine into the DNA of proliferating cells. After removing of the culture medium, the cells were fixed with a hair drier and their DNA was denatured by 200 µl FixDenat for 30 minutes at room temperature. The antibody anti-BrdU-peroxydase was bound (2h at room temperature) to the BrdU incorporated in newly synthesized cellular DNA. The immune complexes were detected with substrate reaction (TMB-TetraMethyl Benzidine). After an acidic stop, the reaction products were quantified by measuring the absorbance at 450 nm by a scanning multiwell spectrophotometer. The developed colour and thereby the absorbance values directly correlate to the amount of DNA synthesis and hereby to the number of proliferating cells in the respective cultures.

Figure 5 shows that the control of cell growth is less efficient with BCG than with BCG Apa⁺⁺.

### 4) Inhibition of FGFR-3 dimerization

T24 and RT112 cells (10⁵/ml) were incubated for 48 h with 10⁶ CFU/ml of BCG or BCG Apa⁺⁺ or without bacteria. The cells were cooled at 4°C during 15 min and incubated for 2 h at 4°C with or without fibroblast growth factor (FGF-1, PROMEGA; 50 ng/ml). After covalent cross-linking with 1 mM bis-(sulfo-succinimidyl)-suberate and 15 mM disuccinimidyl-suberate (PIERCE) for 30 min at room temperature, the cells were lysed, immunoprecipitated with anti-fibroblast growth factor receptor (FGFR-3) C-terminal antibody (C-15 sc-123; SANTA CRUZ BIOTECHNOLOGY INC), and probed on an immunoblot with a polyclonal antibody to the cytoplasmic domain of FGFR-3 (P-18 sc-31162; SANTA CRUZ BIOTECHNOLOGY INC.). The density obtained for the dimers (∼250 kDa) relative to the sum of the density of monomers (∼120 kDa) and dimers were determined.

The dimerisation of FGF receptor (FGFR-3) observed in T24 and RT112 carcinoma cells incubated with FGF is partly inhibited in presence of BCG and totally inhibited in presence of BCG Apa++ (Figures 6 and 7).

### 5) Analysis of MAPK and Akt 1-2 pathways

Analysis of subsequent stimulation pathways was performed. Therefore, T24 cells cultivated in six-well plates were incubated 48 hours with 10⁶ CFU of BCG or BCG Apa⁺⁺ in serum-free DMEM, then 15 minutes with or without EGF (100 ng/ml), and lysed. The presence of phosphorylated mitogen-activated protein kinase p42/p44 (pMAPK) and phosphorylated Akt 1-2 were determined by total protein ELISA by PhosphoDetect ERK1/2 (pThr¹⁸⁵/pTyr¹⁸⁷) (CALBIOCHEM) and PhosphoDetect Akt (pSer⁴⁷³) (CALBIOCHEM) ELISA Kits, respectively.

The results reporting phosphorylation of MAPK (figure 8) and Akt1/2 (figure 9) indicate that the presence of BCG Apa⁺⁺ on the T24 cell surface inhibits the first steps of EGF stimulation and confirm the tendency of BCG Apa⁺⁺ to be more potent than BCG to control the carcinoma cell growth.

### Example 4: Induction of apoptosis pathways in human carcinoma cells by BCG or BCG Apa⁺⁺

Morphological observation of T24 and RT112 human bladder carcinoma cells treated with BCG or BCG Apa⁺⁺ revealed the presence of abundant cytoplasmic vesicles and nuclear abnormalities indicative of apoptosis.

The switch to apoptotic pathways is under the control of the Bcl-2 family of proteins and involves cysteine-aspartic-acid-proteases (caspases), (For a review, see J.M. Adams and S. Cory Oncogene 2007, 26, 1324-1337). Among the Bcl-2 family members, Bcl-2 promotes cell survival and at the opposite, Bid, Bak and Bax promote cell death. Caspases are a group of cysteine proteases, enzymes with a crucial cysteine residue that can cleave other proteins or peptides after an aspartic acid residue. Caspase-8, a protease that recognizes the amino acid sequence IETD (Ile-Glu-Thr-Asp) is a key element in the external apoptosis pathway. Caspase-9, a protease that recognizes the amino acid sequence LEHD (Leu-Glu-His-Asp) is a key element in the internal apoptosis pathway.

Therefore, the activation level of Bcl-2 Bid, Bak, Bax was evaluated in T24 and RT112 cells treated with BCG or BCG Apa++ or untreated, using RNase protection assay (RPA). In addition, caspase-8 and caspase-9 levels were evaluated in T24 cells treated with BCG or BCG Apa++ or untreated, using a colorimetric assay.

### 1) RNase protection assay

The cells (10⁶ per flask) were cultivated with 5.10⁶ CFU of BCG or BCG Apa++ or without bacteria. Twenty-four hours after treatment, the cells were harvested after cold-PBS washes. Cell suspensions were homogenized in TRIzol and RNA was extracted from the lysates using the chloroform-isopropanol method, according to the manufacturer's instruction (INVITROGEN). Dried RNA precipitates were resuspended in DEPC-H₂O (0.1% DiEthylPyroCarbonatein water, INVITROGEN).

mRNA expression was measured by using the RiboQuant multiprobe RNA protection assay (BD BIOSCIENCES PHARMINGEN), following the manufacturer's instructions. Briefly, antisense RNA probes were transcribed using the cDNA template set of Human Apoptosis hAPO-2c (BD BIOSCIENCES PHARMINGEN). For transcription, 1 µl (50 ng) of the template was incubated for 2 h at 37°C in a 19 µl aqueous mixture containing 2 µl of the enzymes (80 U of RNasin and 40 U T7 RNA polymerase), 4 µl of the nucleotide pool (5 mM) containing 3.25 mM Biotin-16-UTP (ROCHE DIAGNOSTICS), 2 µl of DTT (100 mM) and 4 µl of 5x transcription buffer. Except for Biotin-16- UTP, all reagents were supplied by the manufacturer in RiboQuant Non-Rad *In Vitro* Transcription Kit (BD BIOSCIENCES PHARMINGEN). The reaction was terminated by adding 2 µl (2 U) DNase, followed by a 30 min incubation at 37°C. DNase was inactivated by 20 mM EDTA and labeled RNA probes were extracted using Lithium Chloride (LiCl 4 M), followed by ethanol precipitation on dry ice.

For hybridization, 20 µg RNA samples precipitated by ethanol and dried using a vacuum evaporator centrifuge, were resuspended in an 8 µl hybridization buffer (80% formamide, 1 mM EDTA, 400 mM NaCl, and 40 mM Prpes (pre-eazine-N,N'-bis-ethanesulfonic acid; BD BIOSCIENCES PHARMINGEN) at 56°C, mixed with 2 µl (30 ng) of probe prepared as previously described, heated to 90°C, and then incubated at 56°C for 18 h.

For RNase digestion, 10 volumes of a mixture containing RNase A and RNase T1 (100 U) was added and reacted for 45 min at room temperature. After digestion, the samples were mixed with proteinase K and an appropriate buffer supplied in RiboQuant Non-Rad RPA Kit (BD BIOSCIENCES PHARMINGEN) for 15 min at 37°C, after which they were extracted by LiCl and precipitated with ethanol. The samples were then air-dried, resuspended in loading buffer, and size-separated using Polyacrylamide Gel Electrophoresis (PAGE). The bands were electrotransferred to a positively charged nylon membrane (BD BIOSCIENCES PHARMINGEN) by using a Semi-dry Electroblotter (100 mA for 20 min) and crosslinked by UV.

Chemiluminescent probe detection, was performed by using BD RiboQuant Non-Rad Detection kit (BD BIOSCIENCES PHARMINGEN). Nylon membranes were blocked with blocking buffer and conjugated with Streptavidin-Horseradish peroxidase for 15 min at room temperature. Conjugated membranes were washed (supplied Wash Buffer and Substrate Equilibration Buffer) and incubated for 10 min in a freshly prepared equal volume mixture of Stable peroxide solution and Luminol/Enhancer at room temperature. Revealed membranes were exposed to CL-XPosure films (PIERCE) and nucleotide lengths versus migration distances were compared with the standards (30 ng transcribed biotin-labeled probes) on a logarithmic grid. Ribosomal protein L-32 mRNA was used as control.

Figure 10 shows that the high expression of mRNA for Bcl-2 observed in T24 and RT112 cells growing in control cell culture medium was decreased in presence of BCG and disappeared in presence of BCG Apa⁺⁺. At the opposite, messengers for Bax, Bak and Bid that were poorly expressed in cells growing alone, were highly expressed in the presence of BCG and BCG Apa⁺⁺. These results show that BCG Apa⁺⁺ induces with more efficiency the pathways to cell death than BCG.

### 2) Caspase-8 and caspase-9 assays

T24 cells were incubated 6 hours, 12 hours, 24 hours, 36 hours or 48 hours with BCG or BCG Apa++ or without bacteria and lysed. Caspase-8 and caspase-9 were measured on cell lysates using colorimetric assay kits (R&D SYSTEMS). Synthetic peptides (IETD) or (LEHD) conjugated to the chromophore pNA (p-nitroanilide) were added to cell lysates. Upon cleavage of the substrate IEDA-pNA by Caspase-8 or LEHD-pNA by Caspase-9, free pNA is released, resulting in an increase of absorbance at 405 nm, that is measured with a spectrophotometer. Comparison of the absorbance of reaction mixtures made from treated cells with reaction mixtures made from untreated controls allows determination of the increase in caspase activity.

Both caspase-8 (Figure 11) and caspase-9 (Figure 12), expressed as enzymatic activities, were increased in T24 cells growing in presence of BCG and highly enhanced in presence of BCG Apa⁺⁺. The difference between BCG and BCG Apa⁺⁺ were significant (p < 0.05) after 36 h or 48 h, indicating that BCG Apa⁺⁺ is a potent activator of both caspase-8 and caspase-9 activity. These results support the increased apoptosis observed on cells growing in presence of BCG or BCG Apa⁺⁺. In addition, the synergy existing between the caspase-8 and caspase-9 pathways could explain at least in part the increased inhibitory effect on cell growth that was observed with BCG Apa++ (example 3).

### Example 5 : Inhibition of human carcinoma cell growth in vivo by BCG Apa⁺⁺ or BCG

### 1) Animal model

Thirty female Wistar rats (JANVIER), 7 weeks of age at the beginning of the experiment, were used in this study. 24 rats were given 0.05% N-butyl-N-(4-hydroxybutyl)nitrosamine (BBN; KASEI INDUSTRY COMPANY) in the drinking water for 19 weeks. 6 rats were given (normal) drinking water as controls and maintained without any treatment up to 30 weeks. One week after the end of BBN treatment (20^{th} week), the rats received 6 weekly intravesical instillations (0.5 ml) of PBS alone, or PBS containing 10⁸ CFU/ml of BCG or BCG Apa⁺⁺(Figure 13).

### 2) Hemoglobin assay in urine

The urines were harvested at week 30 and their content in haemoglobin measured using the Drabkin's method. This colorimetric assay is based on a measure of cyanmethemoglobin; the total hemoglobin present in urine is rapidly converted to the cyanoderivative at alkaline pH. Drabkin's solution containing alkaline ferricyanide and cyanide reacts with all forms of hemoglobin. 25 µl of the ready to use Hemoglobin reagent from BIOLABO (#3502200, 82250, 82200) was added to 100 µl of rat urines. The mixture was incubated at least 15 minutes at room temperature. The absorbance of sample against blank is read at 540 nm. This non-invasive assay allows to control tumour development and to adjust BCG treatment (dose, frequency of administration) in order to optimize the antitumoral treatment.

The decrease in haemoglobin present in urine observed after BCG therapy was more marked with BCG Apa⁺⁺.

### 3) Clinical observation of bladder

Rats were euthanized at the 30^{th} week (one month after the last BCG treatment), bladders were collected and subjected to a macroscopic examination.

Clinical observation of the bladders indicated the presence of tumors in all the animals of the PBS-treated group and half of the animals of the BCG treated group. By contrast, the bladder was normal in all the animals of the BCG Apa⁺⁺-treated group (Figure 15). BCG Apa⁺⁺ overexpressing APA molecules under mild hypoxia conditions observed in bladder was found to be more active than wild BCG to control carcinoma cell proliferation in bladder of rats receiving a chemical agent promoting carcinoma. In these *in vivo* assays the results were more impressive than *in vitro,* perhaps due to a deeper hypoxia level in urine and bladder than in *in vitro* culture.

### 4) Wild-type p53 expression analysis

Rats were euthanized at the 30^{th} week (one month after the last BCG treatment). Bladders were collected, homogenized and nuclear protein extracts were prepared by Activemotif nuclear extract kit (Active motif, CARLSBAD). Activated wild type p53 were measured by an Enzyme Immunometric Assay Kit (TiterZyme®, EIA900-117, Ann Arbor, MI).

Analysis of wild-type p53 tumor suppressor factor concentration in the tumor cells nucleus, demonstrates no reduction in the BCG Apa⁺⁺ treated group, compared to a 50% reduction in the PBS-treated group and a 25% to 30% reduction in the BCG treated group (Figure 16). The normal value of wild-type p53 present in bladder of BCG Apa⁺⁺ treated rat indicated that the tumoral process was at least decreased or may be switched off at time of bladder harvest.

### Example 6: Transcriptome analysis

RNA extraction was performed as described in example 4. Human cancer pathway finder microarray kits (GEArray^{™} Oligo OHS-033) were obtained from SUPERARRAY BIOSCIENCE CORP.. They included nylon membranes printed by specific cDNA fragments of 113 genes associated with different cancer cascades and classified in six groups (Table II). Probe synthesis and probe biotin labelling were performed by using GEArray AmpoLabeling-LPR Kit (SUPERARRAY BIOSCIENCE CORP.) and Biotin-16-dUTP (ROCHE DIAGNOSTICS), following the manufacturer's instructions. Biotinylated and amplified cDNA probes were hybridized overnight at 60°C with different array membranes and AP-streptavidin chemiluminescent detection was performed by SuperArray Detection Kit (SUPERARRAY BIOSCIENCE CORP.). Membranes were exposed on CL-XPosure films (PIERCE) and image acquisition was accomplished by a desk scanner using 200 dpi. Data acquisition was performed by using the ScanAlyze software version 2.50 (http://rana.lbl.gov/EisenSoftware.htm). Data analysis was completed by GEArray Analyzer software (www.superarray.com). Raw data were subtracted from the mean signals of three negative controls as areas without spotted gene sequences (blanks) or areas spotted by the genes not expressed in human cells (pUC18). For adjusting of loading (1 µg total RNA of stimulated or control T24 cells) these subtracted data were normalized by their ratio to averaged signals of three positive controls (housekeeping genes: β-actin, GAPDH and Ribosomal protein S27a). Additionally, the data were also normalized according to another criterion of lowest signal. Experiment was performed three times to ensure reproducibility of results.

Transcriptome analysis of T24 cells treated with different BCG strains confirms the results from functional studies (examples 3, 4 and 5) and indicate that genes coding for integrins, apoptosis and cell cycle were modulated by BCG and BCG Apa⁺⁺ but not by BCG Apa⁻ (Table I, Table II and figure 17). In addition the modulation is higher with BCG Apa⁺⁺ than with BCG (Table I and figure 17)

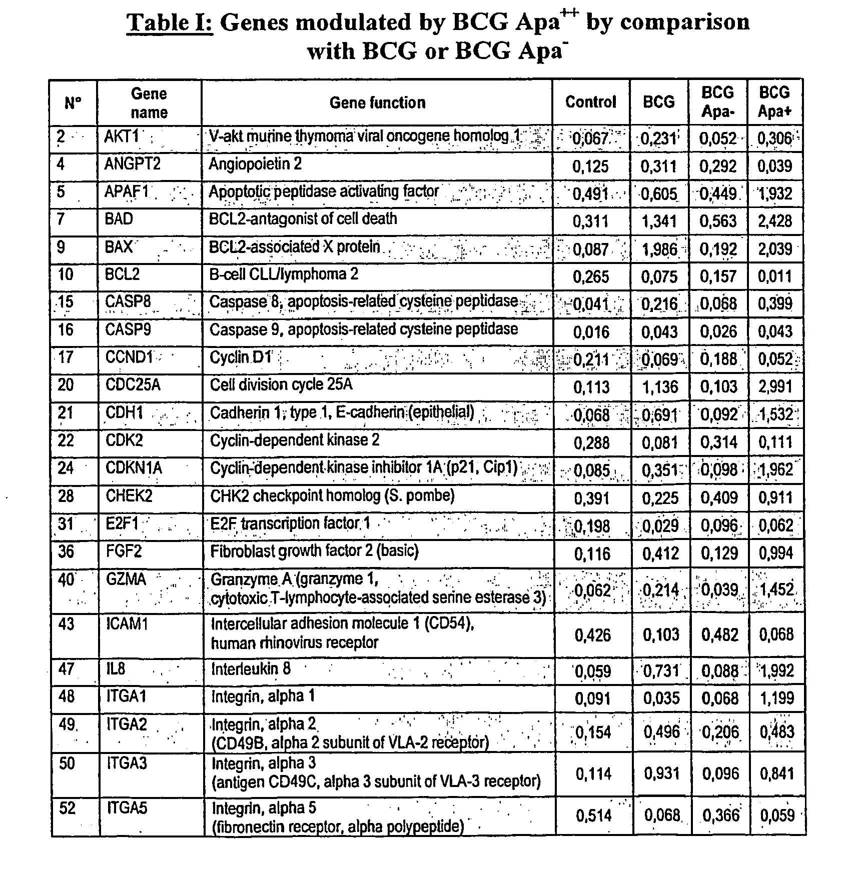

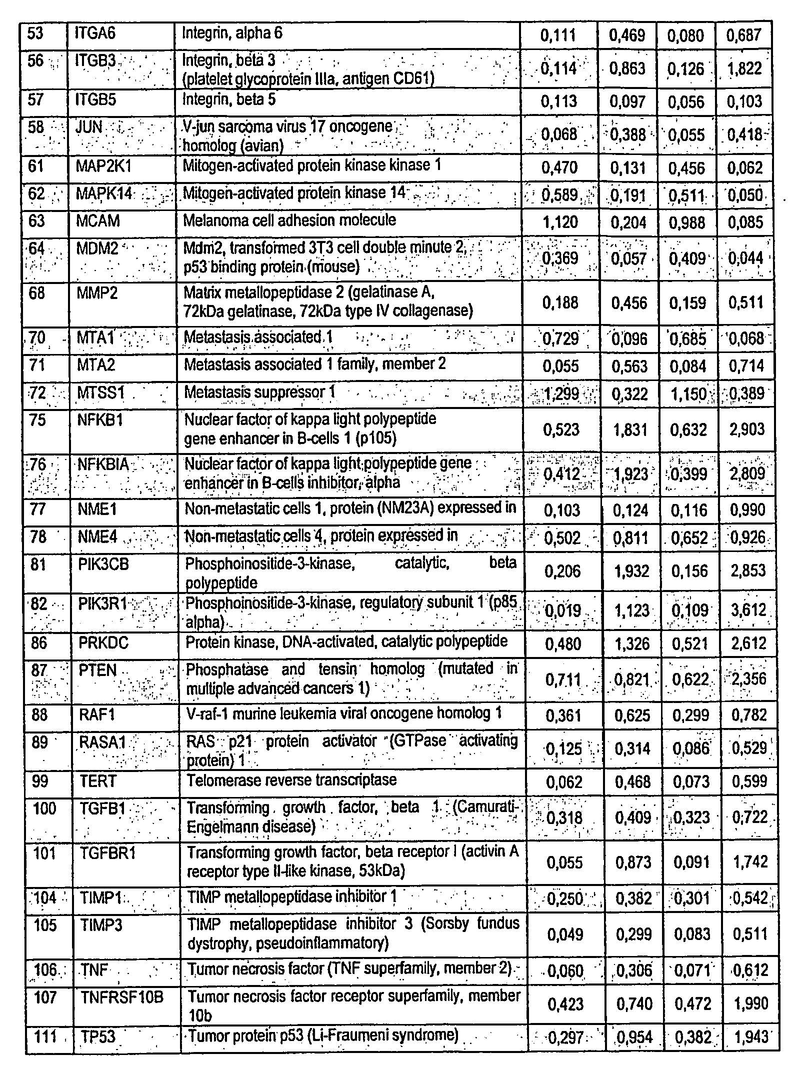

**Table II: Genes affected by BCG Apa⁺⁺ within the different categories of genes analyzed**

| **Gene function** | Gene name* |
|---|---|
| **Cell Cycle Control DNA Damage Repair** | ATM, BRCA1, BRCA2, *CCND1 (cyclin D1),* CCNE1 (cyclin E1), **CDC25A,** *CDK2,* CDK4, **CDKN1A (p21Waf1),** CDKN1B (p27Kip1), CDKN2A (p16Ink4), **CHEK2 (chk2 / Rad53),** *E2F1, MDM2,* **PRKDC (DNA-PK), PTEN,** S100A4, RB1, **TP53 (p53)** |
| **Apoptosis Cell Senescence** | **APAF1, BAD, BAX,** *BCL2,* BCL2L1 (bcl-X), BIRC5 (Survivin), **CASP8, CASP9,** CFLAR (CASPER), **GZMA**, HTATIP2, **TERT (telomerase),** TNFRSF1A (TNF-a receptor), TNFRSF6 (Fas), **TNFRSF10B (DR5),** TNFRSF25 (DR3) |
| **Signal Transduction Molecules Transcription Factors** | **AKT1,** CTNNB1 (β-catenin), ERBB2, ETS2, FOS, **JUN,*MAPK14 (p38 MAPK), MAP2K1* (*MEK),*** MYC, **NFKB1 (NFκB), NFKBIA (IκBα)**, **PIK3CB (PI3K p110b), PIK3R1 (pI3K p85a), RAF1, RASA1,** SNCG, SRC. |
| **Adhesion** | CD44,**CDH1 (E-cadherin),** *ICAM1,* **ITGA1 (integrin a1), ITGA2 (integrin a2), ITGA3 (integrin a3),** ITGA4 (integrin a4), *ITGA5* *(integrin a5),* **ITGA6 (integrin a6),** ITGAV (integrin aV), ITGB1 (integrin β1), **ITGB3** **(integrin β3),** *ITGB5 (integrin β5), MCAM,* MICA (MUC18), *MTSS1,* NCAM1, PNN, SYK, UCC1. |
| **Angiogenesis** | ANGPT1 (angiopoietin-1), *ANGPT2 (angiopoietin-2),* BAI1, COL18A1 (endostatin), EGF, EGFR, **FGF2 (bFGF),** FGFR2, FLT1 (VEGFR), HGF, IFNA1 (IFNa), IFNB1 (IFN?), IGF1, **IL8**, PDGFA, PDGFB, TEK (tie-2), **TGFB1, TGFBR1 (ALK-5),** THBS1 (thrombospondin-1), THBS2 (thrombospondin-2), **TNF,** VEGF. |
| **Invasion Metastasis** | KISS1, KAI1, MET, MMP1 (collagenase-1), **MMP2 (gelatinase A),** MMP9 (gelatinase B), *MTA1*, **MTA2, NME1, NME4 (Nm23),** PLAU, PLAUR, S100A4, SERPINB2 (PAI2), SERPINB5 (maspin), SERPINE1 (PAI1), **TIMP1, TIMP3,** TWIST1. |

| | |
|---|---|
| *genes modulated by BCG Apa++ have been underlined: Induction is in bolded fonts and Suppression is in italic fonts. | |

## Claims

1. A recombinant vector, **characterized in that** it comprises a mycobacterial FAP protein coding sequence under the transcriptional control of a promoter sequence that is specifically up-regulated under hypoxic conditions.

2. The recombinant vector according to claim 1, **characterized in that** said mycobacterial FAP protein is the *Mycobacterium tuberculosis* APA protein.

3. The recombinant vector according to claim 1 or claim 2, **characterized in that** said promoter is a mycobacterial promoter.

4. The recombinant vector according to claim 3, **characterized in that** said promoter is selected from the group consisting of the hspX promoter and the TB31.7 promoter.

5. The recombinant vector according to anyone of claims 1 to 4, which is a plasmid.

6. An expression cassette derived from the recombinant vector according to anyone of claims 1 to 5, **characterized in that** it is a polynucleotide fragment comprising the promoter sequence which is specifically up-regulated under hypoxic conditions, as defined in any one of claims 1, 3 and 4, operatively linked to the mycobacterial FAP protein coding sequence, as defined in claim 1 or claim 2.

7. A host cell which is genetically modified by a recombinant vector according to anyone of claims 1 to 5.

8. A host cell which is genetically modified and has the expression cassette according to claim 6 integrated in its genome.

9. The host cell according to claims 7 or 8, **characterized in that** it is a recombinant mycobacteria strain.

10. The host cell according to claim 9, **characterized in that** it is derived from a mycobacteria selected from the group consisting of: *Mycobacterium bovis* strain BCG, *Mycobacterium smegmatis, Mycobacterium fortuitum* and *Mycobacterium microti.*

11. The host cell according to claim 10, **characterized in that** it is a recombinant *Mycobacterium bovis* BCG strain, stably transformed with the plasmid pYAPA2031, deposited at the Collection Nationale de Cultures de Microorganismes, 25 rue du Docteur Roux, 75724 Paris Cedex 15, on July 21 2006, under accession number I-3659.

12. An antitumoral composition, **characterized in that** it comprises a suitable amount of a recombinant vector according to anyone of claims 1 to 5 or a host cell according to anyone of claims 7 to 11, in an acceptable carrier.

13. The antitumoral composition according to claim 12, **characterized in that** it comprises living recombinant mycobacteria.

14. The antitumoral composition according to claim 12, **characterized in that** it comprises killed recombinant mycobacteria.

15. The antitumoral composition according to claim 14, **characterized in that** said killed recombinant mycobacteria are extended freeze-dried recombinant mycobacteria.

16. The antitumoral composition according to anyone of claims 12 to 15, **characterized in that** it comprises between 10⁶ and 10¹⁰ CFU or CFU equivalent of recombinant mycobacteria.

17. The antitumoral composition according to anyone of claims 13 to 16, **characterized in that** it comprises an immunostimulatory agent.

18. Use of a recombinant vector according to anyone of claims 1 to 5 or a host cell according to anyone of claims 7 to 11 for the manufacture of a medicament intended for the prevention or the treatment of a tumor in a subject.

19. The use according to claim 18, **characterized in that** said medicament is intended for the treatment of epithelial tumors selected from the group consisting of: transitional cell carcinoma of the bladder, lung carcinoma, cervix carcinoma and colon carcinoma.

## Patentansprüche

1. Rekombinanter Vektor, **dadurch gekennzeichnet, dass** er eine ein mycobakterielles FAP-Protein codierende Sequenz unter der transkriptionellen Kontrolle einer Promotorsequenz, welche unter hypoxischen Bedingungen hochreguliert ist, umfasst.

2. Rekombinanter Vektor gemäß Anspruch 1, **dadurch gekennzeichnet, dass** das mycobakterielle FAP-Protein das *Mycobakterium* tuberculosis-APA-Protein ist.

3. Rekombinanter Vektor gemäß Anspruch 1 oder Anspruch 2, **dadurch gekennzeichnet, dass** der Promotor ein mycobakterieller Promotor ist.

4. Rekombinanter Vektor gemäß Anspruch 3, **dadurch gekennzeichnet, dass** der Promotor aus der Gruppe, bestehend aus dem hspX-Promotor und dem TB31.7-Promotor, ausgewählt ist.

5. Rekombinanter Vektor gemäß einem der Ansprüche 1 bis 4, der ein Plasmid ist.

6. Expressionskassette, die aus dem rekombinanten Vektor gemäß einem der Ansprüche 1 bis 5 stammt, **dadurch gekennzeichnet, dass** sie ein Polynucleotidfragment ist, das die Promotorsequenz, welche unter hypoxischen Bedingungen spezifisch hochreguliert wird, wie sie in einem der Ansprüche 1, 3 und 4 definiert ist, funktionell verknüpft mit der ein mycobakterielles FAP-Protein codierenden Sequenz, wie sie in Anspruch 1 oder Anspruch 2 definiert ist, umfasst.

7. Wirtszelle, die durch einen rekombinanten Vektor gemäß einem der Ansprüche 1 bis 5 genetisch modifiziert ist.

8. Wirtszelle, die genetisch modifiziert ist und die Expressionskassette gemäß Anspruch 6 in ihr Genom integriert hat.

9. Wirtszelle gemäß Anspruch 7 oder 8, **dadurch gekennzeichnet, dass** sie ein rekombinanter Mycobakterien-Stamm ist.

10. Wirtszelle gemäß Anspruch 9, **dadurch gekennzeichnet, dass** sie von einem Mycobakterium stammt, das aus der Gruppe, bestehend aus *Mycobakterium bovis*-Stamm BCG, *Mycobacterium smegmatis, Mycobacterium fortuitum* und *Mycobacterium microti,* ausgewählt ist.

11. Wirtszelle gemäß Anspruch 10, **dadurch gekennzeichnet, dass** sie ein rekombinanter *Mycobakterium bovis-*BCG Stamm ist, stabil transformiert mit dem Plasmid pYAPA2031, hinterlegt bei der Collection Nationale de Cultures de Microorganismes, 25 rue du Docteur Roux, 75724. Paris Cedex 15, am 21. Juli 2006 unter der Eingangsnummer I-3659.

12. Antitumorale Zusammensetzung, **dadurch gekennzeichnet, dass** sie eine geeignete Menge eines rekombinanten Vektors gemäß einem der Ansprüche 1 bis 5 oder eine Wirtszelle gemäß einem der Ansprüche 7 bis 11 in einem annehmbaren Träger umfasst.

13. Antitumorale Zusammensetzung gemäß Anspruch 12, **dadurch gekennzeichnet, dass** sie lebende rekombinante Mycobakterien umfasst.

14. Antitumorale Zusammensetzung gemäß Anspruch 12, **dadurch gekennzeichnet, dass** sie abgetötete rekombinante Mycobakterien umfasst.

15. Antitumorale Zusammensetzung gemäß Anspruch 14, **dadurch gekennzeichnet , dass** die abgetöteten rekombinanten Mycobakterien ausgedehnt gefriergetrocknete rekombinante Mycobakterien sind.

16. Antitumorale Zusammensetzung gemäß einem der Ansprüche 12 bis 15, **dadurch gekennzeichnet, dass** sie zwischen 10⁶ und 10¹⁰ KbE oder KbE-Äquivalent rekombinante Mycobakterien umfasst.

17. Antitumorale Zusammensetzung gemäß einem der Ansprüche 13 bis 16, **dadurch gekennzeichnet, dass** sie ein immunstimulatorisches Agens umfasst.

18. Verwendung eines rekombinanten Vektors gemäß einem der Ansprüche 1 bis 5 oder einer Wirtszelle gemäß einem der Ansprüche 7 bis 11 für die Herstellung eines Medikaments zum Zweck der Prävention oder der Behandlung eines Tumors in einem Subjekt.

19. Verwendung gemäß Anspruch 18, **dadurch gekennzeichnet, dass** das Medikament zur Behandlung von epithelialen Tumoren, ausgewählt aus der Gruppe, bestehend aus Übergangsepithelkarzinom der Blase, Lungenkarzinom, Cervixkarzinom und Colonkarzinom, gedacht ist.

## Revendications

1. Vecteur recombinant, **caractérisé en ce qu'**il comprend une séquence codant pour une protéine FAP mycobactérienne sous le contrôle transcriptionnel d'une séquence de promoteur qui est spécifiquement régulée positivement dans des conditions d'hypoxie.

2. Vecteur recombinant selon la revendication 1, **caractérisé en ce que** ladite protéine FAP mycobactérienne est la protéine APA de *Mycobacterium tuberculosis.*

3. Vecteur recombinant selon la revendication 1 ou la revendication 2, **caractérisé en ce que** ledit promoteur est un promoteur mycobactérien.

4. Vecteur recombinant selon la revendication 3, **caractérisé en ce que** ledit promoteur est sélectionné dans le groupe constitué par le promoteur hspX et le promoteur TB31.7.

5. Vecteur recombinant selon l'une quelconque des revendications 1 à 4, **caractérisé en ce qu'**il s'agit d'un plasmide.

6. Cassette d'expression dérivée du vecteur recombinant selon l'une quelconque des revendications 1 à 5, **caractérisée en ce qu'**il s'agit d'un fragment de polynucléotide comprenant une séquence de promoteur qui est spécifiquement régulée positivement dans des conditions d'hypoxie, telle que définie à l'une quelconque des revendications 1, 3 et 4, fonctionnellement liée à une séquence codant pour une protéine FAP mycobactérienne, telle que définie à la revendication 1 ou à la revendication 2.

7. Cellule hôte génétiquement modifiée par un vecteur recombinant selon l'une quelconque des revendications 1 à 5.

8. Cellule hôte génétiquement modifiée et ayant une cassette d'expression selon la revendication 6 intégrée dans son génome.

9. Cellule hôte selon la revendication 7 ou 8, **caractérisée en ce qu'**il s'agit d'une souche mycobactérienne recombinante.

10. Cellule hôte selon la revendication 9, **caractérisée en qu'**elle est dérivée d'une mycobactérie sélectionnée dans le groupe constitué par : une souche de *Mycobacterium bovis* BCG, *Mycobacterium smegmatis, Mycobacterium fortuitum* et *Mycobacterium microti.*

11. Cellule hôte selon la revendication 10, **caractérisée en ce qu'**il s'agit d'une souche de *Mycobacterium bovis BCG* recombinante, transformée de façon stable avec le plasmide pYAPA2031, déposée auprès de la Collection Nationale de Cultures de Microorganismes, 25 rue du Docteur Roux, 75724 PARIS Cedex 15, le 21 juillet 2006, sous le numéro d'accès I-3659.

12. Composition antitumorale, **caractérisée en ce qu'**elle comprend une quantité appropriée du vecteur recombinant selon l'une quelconque des revendications 1 à 5 ou d'une cellule hôte selon l'une quelconque des revendications 7 à 11, dans un véhicule acceptable.

13. Composition antitumorale selon la revendication 12, **caractérisée en ce qu'**elle comprend des mycobactéries recombinantes vivantes.

14. Composition antitumorale selon la revendication 12, **caractérisée en ce qu'**elle comprend des mycobactéries recombinantes tuées.

15. Composition antitumorale selon la revendication 14, **caractérisée en ce que** lesdites mycobactéries recombinantes tuées sont des mycobactéries recombinantes lyophilisées de manière extensive.

16. Composition antitumorale selon l'une quelconque des revendications 12 à 15, **caractérisée en ce qu'**elle comprend entre 10⁶ et 10¹⁰ UFC ou équivalents d'UFC de mycobactéries recombinantes.

17. Composition antitumorale selon l'une quelconque des revendications 13 à 16, **caractérisée en ce qu'**elle comprend un agent immunostimulant.

18. Utilisation d'un vecteur recombinant selon l'une quelconque des revendications 1 à 5 ou d'une cellule hôte selon l'une quelconque des revendications 7 à 11, pour la préparation d'un médicament destiné à la prévention ou au traitement d'une tumeur chez un sujet.

19. Utilisation selon la revendication 18, **caractérisée en ce** ledit médicament est destiné au traitement des tumeurs épithéliales sélectionnées dans le groupe constitué par : les carcinomes à cellules transitionnelles de la vessie, les carcinomes du poumon, les carcinomes du col de l'utérus et les carcinomes du côlon.
